# EUROPEAN PATENT SPECIFICATION

(11) **EP 0 532 668 B1**
(45) Date of publication and mention of the grant of the patent: **15.11.1995**
(21) Application number: 91912140.0
(22) Date of filing: 17.05.1991
(51) Int. Cl.: C07H 17/02, A61K 31/70

(54) **NOVEL ALPHA-GLUCOSIDASE INHIBITORS**
NEUE ALPHA-GLUKOSIDASEINHIBITOREN
NOUVEAUX INHIBITEURS D'ALPHA-GLUCOSIDASE

(30) Priority: 08.06.1990 EP 90401582
(43) Date of publication of application: 24.03.1993
(73) Proprietor: MERRELL DOW PHARMACEUTICALS INC., Cincinnati Ohio 45215-6300 (US)
(72) Inventor: DUCEP, Jean-Bernard, F-68280 Sundhoffen (FR); DANZIN, Charles, F-67000 Strasbourg (FR)
(74) Representative: Gillard, Marie-Louise
(86) International application number: US9103474
(87) International publication number: WO9118915

(56) References cited:
- EP-A- 0 262 404
- DE-A- 3 712 799
- GB-A- 2 088 365
- GB-A- 2 181 729

## Description

This invention relates to novel polyglycosidyl derivatives of 1-deoxy-nojirimycin, to the processes for their preparation and to their end-use applications, particularly as to their use in the treatment of diabetes.

More specifically this invention relates to novel polyglycosyl derivatives of 1-deoxy-nojirimycin, to the chemical processes for their preparation, to their a-glucosidase inhibiting properties, and to their end-use application in the treatment of diabetes, obesity and those diseases associated with retroviruses, particularly the HIV virus reported to be causative of acquired immune deficiency syndrome (AIDS).

UK Patent Application GB 2 181 729 discloses glucosylmoranoline derivatives useful as remedies for diabetes mellitus.

European Patent Application 0 262 404 discloses glucosylmoranoline derivatives useful as a remedy for diabetes.

Still more specifically this invention relates to the novel 1-deoxy nojirimycin derivatives of the formula I 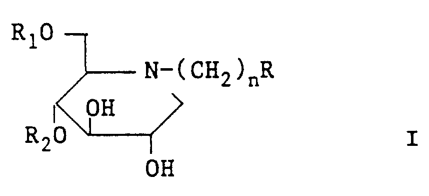 and the pharmaceutically acceptable acid addition salts thereof wherein
n is zero, 1 or 2,
R is a glycosyl moiety containing 1 to 3 hexose or pentose units, said units optionally bearing an ether or acyl radical at the anomeric carbon atom of the terminal hexose or pentose unit, and one of R₁ and R₂ is H and the other is a-D-glucopyranosyl. The glycosyl moiety represented by "R" in Formula I are radicals which contain from 1 to 3 hexose or pentose units which optionally bear an ether or an acyl radical at the anomeric carbon atom of the terminalhexose or pentose moiety.

Acid addition salts are those salts formed with such inorganic acids as, for example, hydrochloric, hydrobromic, sulfuric, phosphoric and like acids; with organic carboxylic acids such as, for example, acetic, propionic, glycolic, lactic, pyruvic, malonic, succinic, fumaric, tartaric, citric, ascorbic, maleic, hydroxymaleic and dihydroxymaleic, benzoic, 2-acetoxybenzoic, mandelic and like acids; and with organic sulfonic acids such as methanesulfonic acid and p-toluenesulfonic acid.

In general, the mono-, di- or trisaccharide moiety (i.e., the glycosyl moiety defined by R) may be attached directly - or thru a (CH₂)ₙ alkylene bridge - to the nitrogen atom of the 1-deoxynojirimycin moiety thru either an exocyclic or ring carbon atom of the pentose or hexose ring thereby forming a variety of position isomers for each individual glycosyl moiety. Also, similar or dissimilar pentose or hexose moieties may be linked to each other thru a glycosidic oxygen bridge wherein the bridging oxygen atom is attached to an exocylic and/or endocyclic carbon atom of the pentose or hexose moiety of which the glycosyl radical is comprised; again the position isomers all being contemplated as being within the scope of this invention.

Exemplary of glycosyl radicals contemplated by the "R" designation in Formula I are such monosaccharides as 6- or 4-glucosyl, 6- or 4-galactosyl, 4-fucosyl, 1-, 2- or 6-fructosyl, 6- or 4-mannosyl, 4-ribosyl, 4-arabinosyl, 4-xylosyl, 6- or 4-allosyl, 6- or 4-altrosyl, 6- or 4-gulosyl, 6- or 4-idosyl, 6- or 4-talosyl and 4- lyxosyl, such disaccharides as 4- or 6-isomaltosyl, 4- or 6-trehalosyl, ;8-4- or 6-cellobiosyl, maltosyl, and such trisaccharides as maltotriosyl and cellotriosyl. Preferred glycosyl radicals are 6- or 4-glucosyl, 1- or 6- fructosyl, 6- or 4-maltosyl and 6- or 4-isomaltosyl. Ether derivatives are those derivatives wherein the hydroxyl group attached to the anomeric carbon atom is etherified and include the C₁ -₈ alkyl derivatives, preferably methyl, ethyl, propyl, butyl, pentyl, hexyl, cyclohexyl, cyclohexylmethyl, t-butyl, isobutyl, isopropyl and aromatic derivatives such as phenyl and benzyl and the like. Acyl derivatives, such as those formed at the anomeric carbon atom by reaction of the free hydroxy radical with C₁ -₈ alkanoic acids or benzoic acids, are also contemplated even though such acylated moieties may easily be removed from the glycosyl radical. Preferred acyl radicals are those formed with acetic or benzoic acids, although acyl radicals formed from such acids as propionic, n-butyric, isobutyric, n-valeric, hexanoic and phenylacetic acid are contemplated.

The a-D-glucopyranosyl moiety of R₁ and R₂ has the structural formula 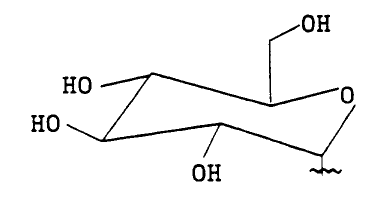 the wavy line indicating the bond by which it is attached to the oxygen of either the 4- or 6- positions of the 1-deoxynojirimycin moiety.

The final products of formula I may be prepared by one of two alternate methods which are depicted by Reaction Scheme A and B. Reaction A involves the condensation of a 1-halo (preferably bromo)-2,3,4-trihydroxy-protected (preferably benzyl)-6-O-t-butyldimethylsilyl-a-D-glucopyranosyl derivative with an N-R'- substituted -2,3,4-trihydroxy-protected (preferably benzyl)-6-O-hydroxyl-I-deoxynojirimycin derivative (R' being an OH-protected glycosyl moiety as previously defined) to produce the expected hydroxy-protected analogs of formula I which are then deprotected to produce the desired compounds embraced by formula I. Reaction Scheme B involves the condensation reaction of a halo derivative of a glycosyl with a 1-deoxynojirimycin bearing an a-D-glucopyranosyl moiety to produce the desired compounds of formula I. In essence, Reaction Scheme A illustrates the preparation of compounds of formula I wherein the a-D-glucopyranosyl moiety is attached to the 1-deoxynojirimycin moiety via the 6-position linkage (i.e., R₁ is a-D-glucopyranosyl and R₂ is H), whereas Reaction Scheme B illustrates the preparation of compounds of formula I wherein the a-D-glucopyranosyl moiety is attached to the 1-deoxynojirimycin moiety via the 4- position linkage (i.e., R₂ is a-D-glucopyranosyl and R₁ is H)

For convenience, and to better teach the preparation of the compounds of this invention, Reaction Schemes A and B depict specific reactants. However, it is to be understood that the schemes have general applicability to the various glycosyl moieties (as defined by R on formula I), it being understood to those of ordinary still in the art that the hydroxyl groups of any of the R-glycosyl moieties will be suitably protected and deprotected as indicated for the specific reactants used in the Reaction Schemes shown below. 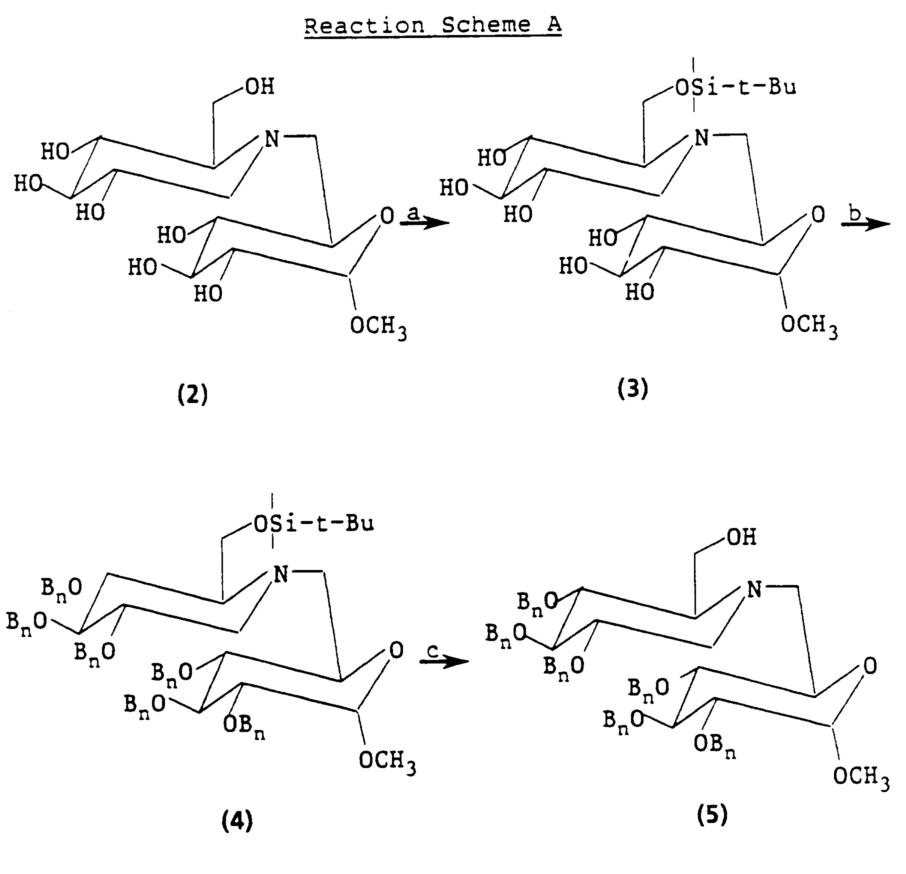 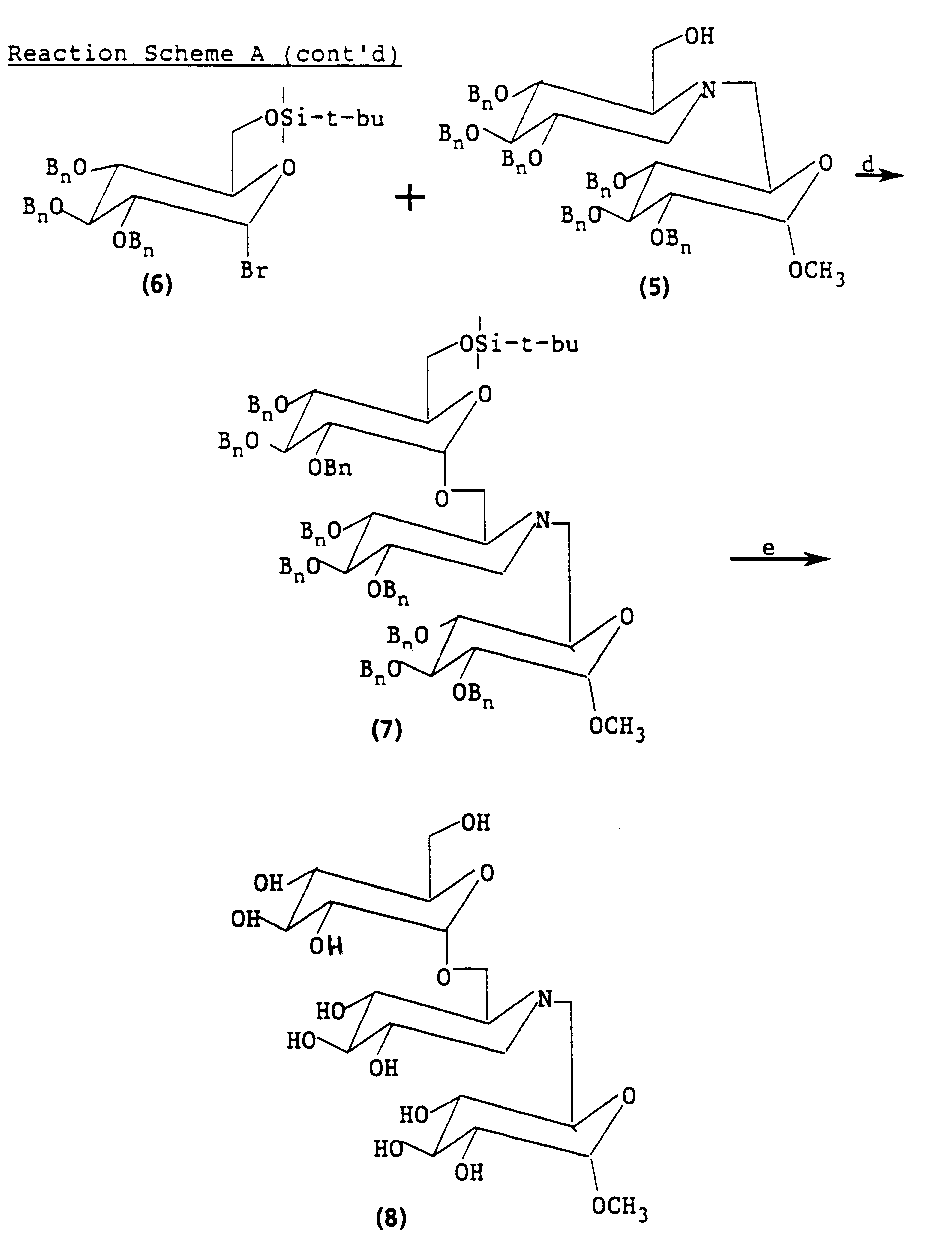 wherein Bₙ is a benzyl or equivalently functioning hydroxy-protecting group and t-Bu is tertiary butyl. 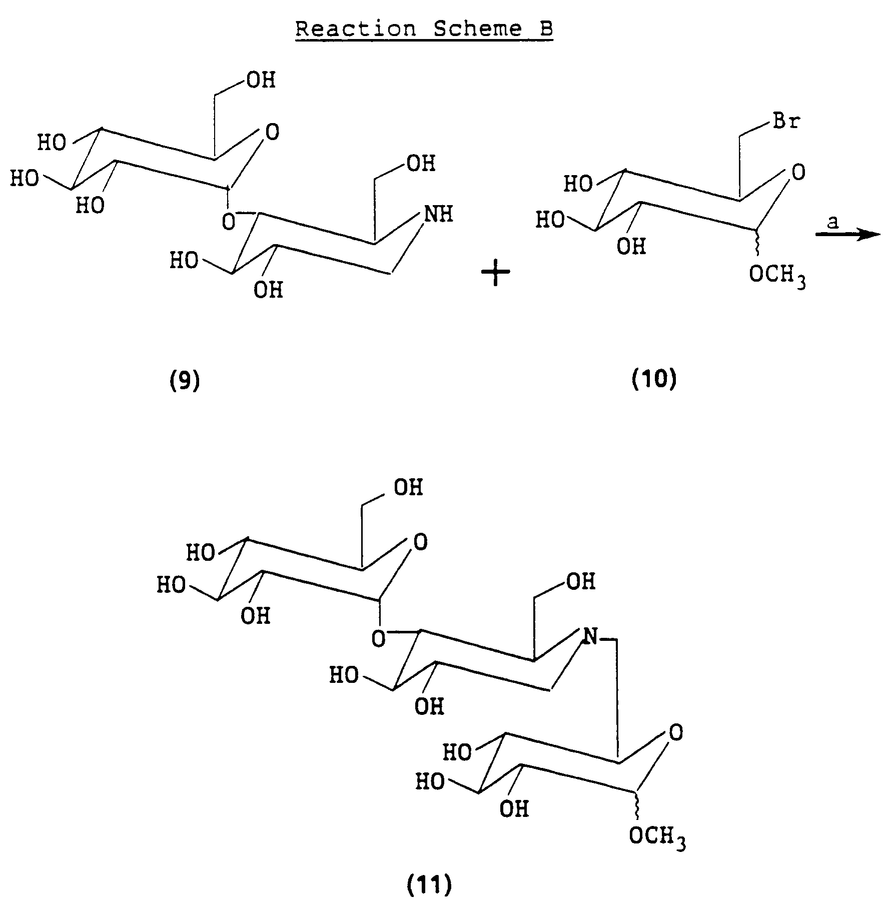 wherein the wavy line of formula 10 and 11 indicates an a- or β-methyl glycoside at the anomeric carbon position (or the a- and β-free hydroxy analog of the glycoside moiety).

In effecting Reaction Scheme A, step (a) entails converting the 6-hydroxy moiety of 1,5-dideoxy-1,5-[(6-deoxy-1-O-methyl-6-a-D-glucopyranosyl)imino]D-glucitol (2) to its O-t-butyldimethylsilyloxy analog (3) by reaction with t-butyldimethylchlorosilane in the presence of imidazole in dimethylformamide. Step (b) converts (3) to its benzylated analogs (4) by reaction with 6 equivalents of benzyl bromide and 6 equivalents of sodium hydride in the presence of n-tetrabutyl ammonium iodide in dimethylformamide at room temperature, preferably overnight and the resulting products hydrolyzed with aqueous ammonium chloride to the desired products which, when subjected to step (c) involving the reaction with n-tetrabutyl ammonium fluoride trihydrate in tetrahydrofuran yields compounds (5). Step (d) involves the reaction of compounds (5) with 2,3,4-tri-O-benzyl-6-0-t-butyldimethylsilyl-a-D-glucopyranosyl bromide (compounds 6) in the presence of diisopropylethylamine in ethanol-free chloroform at room temperature to produce compounds (7) which are then deprotected by standard debenzylation techniques such as catalytic hydrogenation in ethanol using palladium on carbon until up take of hydrogen is complete (about 7 days) or by transfer hydrogenation using formic acid in methanol with Pd/C. When transfer hydrogenation techniques are utilized the products are in the form of their ammonium salts and must be neutralized, preferably using ion exchange resins, such as Dowex AG 1X8 O He form.

In effecting Reaction Scheme B, the 1-deoxy nojirimycin derivative (9) is reacted with the appropriate bromo derivative of an R-glycosyl moiety, e.g. compound (10), in dimethylformamide at about 80 _{°} C for about 48 hours to produce the desired compounds (11).

In general the starting materials such as compounds (2) are known compounds. In those instances wherein the 1-deoxy nojirimycin reactants analogous to compounds of formulae 2,3,4 and 5 which contain a N-glycosyl moiety (i.e. R is glycosyl) other than that shown in formulae 2, 3, 4, 5, 7, 8, 10 and 11, such compounds may also be prepared by methods analogously known in the art. It is preferred to condense an appropriately hydroxy protected 1-deoxy-nojirimycin (2) with an appropriately hydroxy-protected activated glycosyl moiety, preferably using a triflate or halide, preferably the iodide, but including bromide and chloride and including mesylates or tosylates or other equivalently functioning moieties appreciated by those of ordinary skill in the art. In those instances wherein the 1-deoxy-nojirimycin is coupled with a triflate the reaction is effected by refluxing an admixture of equimolar quantities of the reactants in an alcohol- and water-free solvent, preferably a chlorinated solvent such as chloroform, under an inert atmosphere, preferably under nitrogen or argon, for about 1 to 3 days until the reaction is completed. Following standard procedures for the isolation and purification of the reaction products, the protecting groups are removed to obtain the desired product. Debenzylation is readily effected with standard techniques such as catalytic hydrogenation in an appropriate solvent, e.g. ethanol, using a catalyst such as palladium on carbon, or by transfer hydrogenation using cyclohexene and methanol. In those instances wherein esters are utilized (partially or completely) as the hydroxy protecting groups, it is preferred to first remove the ester group by treatment with an alkali alkoxide, e.g. sodium methoxide in methanol to hydrolyze the esters and then deprotect the benzyl ethers using the foregoing hydrogenation procedures.

In those instances wherein a glycosyl halide is coupled with the 1-deoxy-nojirimycin the reaction is effected by heating the appropriately hydroxy protected reactants in dry dimethylformamide (DMF) or other equivalently functioning solvent, at about 60 _{°} -90 _{°} C for about 12 to 36 hours, said heating taking place using excess amounts of a weak base (K₂C0₃) or a molecular sieve, preferably using excess molar amounts of the halide (up to three times) relative to the amine.

The foregoing reactions are illustrated by the following reaction schemes C and D. 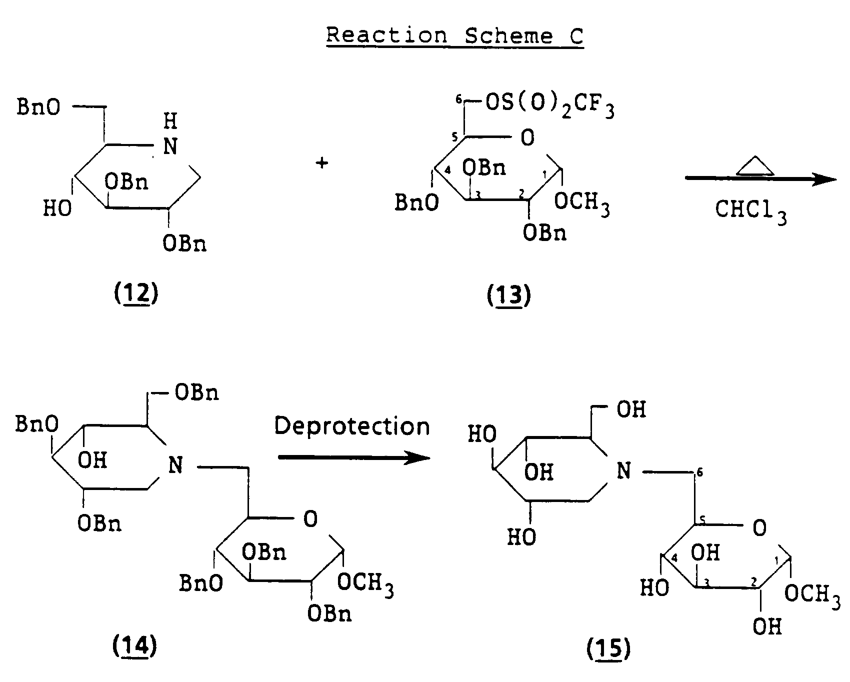 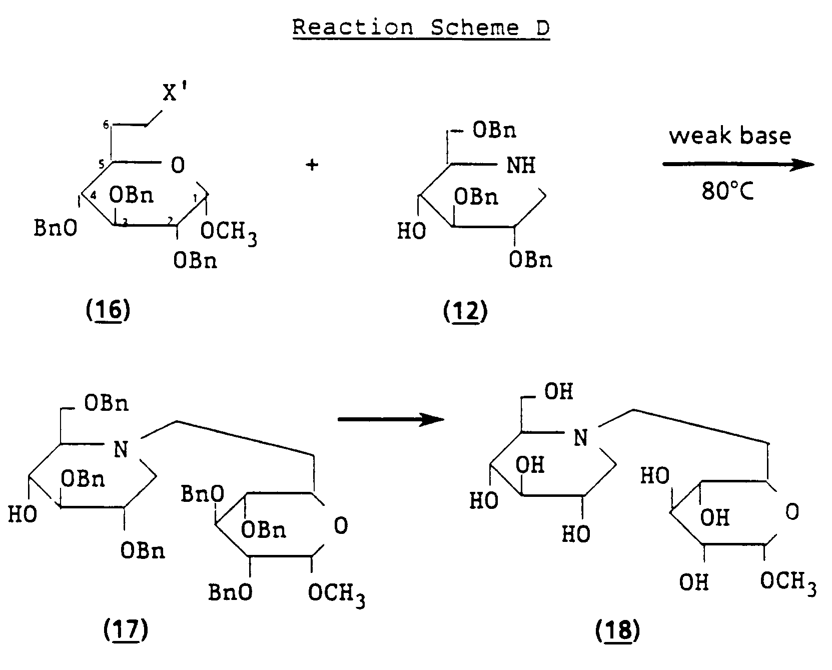 Otherwise depicted, the reaction scheme may more generally be depicted by the following reaction scheme 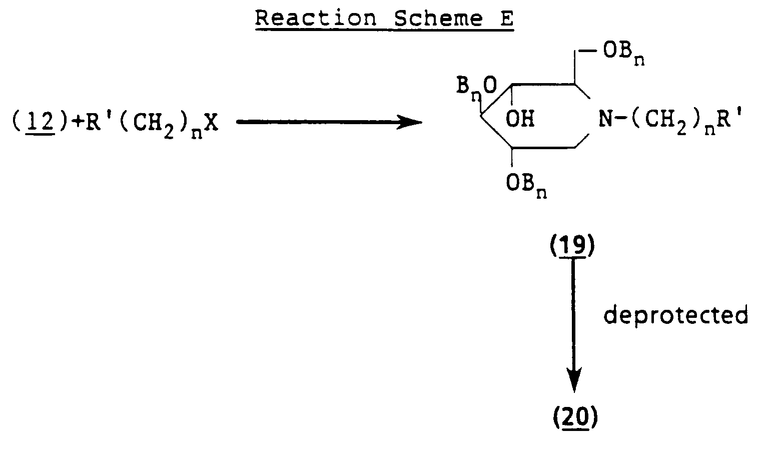 wherein X is a halide (preferably iodide) or a triflate, n is zero, one or two and R' is a glycosyl moiety (as defined by Formula I) having its OH groups protected with a benzyl protecting group, and Bn is benzyl and compound (12) is as depicted in Reaction Schemes C and D.

Appropriately hydroxy protected glycosyl halides (16) and triflates (13) are those glycosyl radicals (mono-, di- or trisaccharides of Formula I) where in the hydroxy groups have been protected with an ester or ether moiety. Preferred esters are the acetate or benzoate esters although other alkanoyl esters, particularly those containing up to six carbon atoms, may be used. The preferred ether is the benzyl ether. Such protected compounds may be prepared by standard procedures very well known and understood in the art.

The glycosyl triflates (of which compound 13 is representative) are prepared by standard procedures such as by reaction of an hydroxy protected glycosyl with trifluoromethylsulfonate anhydride in a chlorinated solvent with pyridine for about 1-3 hours at about -78 _{°} C to -10°C. (It is to be noted that the anomeric carbon atom which optionally may be etherified or acylated is that carbon atom at the 1-position of the compound of Formula 13, said carbon atom bearing an ether derivative.)

The glycoside halides (of which compound 16 is representative) may be prepared by standard techniques starting with an appropriately hydroxy protected glycoside bearing one free hydroxy group. In these instances the alcohol is converted to its aldehyde by a Swern oxidation (treatment with oxalyl chloride in dimethylsulfoxide and triethylamine) followed by an in situ conversion of the aldehyde to an olefin by a Wittig reaction (going through a "ylide" prepared from methyltriphenylphosphonium bromide using one equivalent each of n-butyllithium, potassium t-butoxide and t-butanol in tetrahydrofuran at room temperature for about 4 to 8 hours). The olefin is converted to its corresponding alcohol by hydroboration (treatment with borondimethylsulfide, under nitrogen, followed by oxidation with hydrogen peroxide and sodium hydroxide). The alcohol is mesylated (treatment with mesyl chloride in CH₂CI₂ in excess NEt₃ at -15 °C to 0° C) and the mesylate converted to its halide (by treatment in ether at 0 _{°} C with magnesium halide), preferably using the iodide.

The 1-deoxy-nojirimycin is prepared by reducing the corresponding 6-lactam of 2,3,6-tribenzyloxy-D-gluconic acid with boron dimethylsulfide followed by treatment with gaseous hydrochloric acid.

The following examples illustrate the processes and techniques suitable for the preparation of the compounds of this invention.

### EXAMPLE 1

Preparation of 1,5-DIDEOXY-4-O-(α-D-GLUCOPYRANOSYL)-1,5-[(6-DEOXY-1-O-METHYL-6-α-D-GLUCOPYRANOSYL)IMINO]-D-GLUCITOL

1,5-Dideoxy-4-O-(a-D-glucopyranosyl)-1,5-imino -D-glucitol [Y. Ezure, Agric. Biol. Chem., 49, 2159, (1985)] (0.325 g, 1 mmol) and methyl 6-bromo-6-deoxy-a-D-glucopyranoside [R. L. Whistler and A. K. M. Anisuzzaman, Methods Carbohydr. Chem., 8, 227, (1980)] (0.282 g, 1.1 mmol) are dissolved in dimethyl formamide (5 ml) and heated during 48 hours at 80 °C. Dimethyl formamide is evaporated under high vacuum. The residue is taken with water and neutralized with amberlyst A26 OH^{e} form (3 g) and filtered. Water is evaporated under vacuum. Flash chromatography on silica gel and elution with a 50:50:4 mixture of methanol, chloroform and water afforded the expected compound 1,5-dideoxy-4-0-(a-D-glucopyranosyl)-1,5-[(6-deoxy-1-0-methyl-6-a-D-glucopyranosyl)imino]-D-glucitol as an amorphous solid (145 mg, 30%).

### EXAMPLE 2

Preparation of 6-O-t-BUTYLDIMETHYLSILYL-1,5-DIDEOXY-1,5-[(6-DEOXY-1-O-METHYL-6-α-D-GLUCOPYRANOSYL)IMINO]-D-GLUCITOL

1,5-Dideoxy-1,5-[(6-deoxy-1-O-methyl-6-α-D-glucopyranosyl)-imino]-D-glucitol [European Patent Application EP 0 344 383 A1, published December 6, 1989] (0.5 g, 1.47 mmol) is dissolved in dry dimethyl formamide (5 ml). Imidazole (0.23 g, 3.39 mmol) and t-butyldimethylchlorosilane (0.226 g, 1.5 mmol) are added. The mixture is stirred 48 hours at room temperature. Dimethyl formamide is evaporated under high vacuum. Flash chromatography on silica gel and elution with methanol affords the expected product 6-O-t-butyldimethylsilyl-1,5-dideoxy-1,5-[(6-deoxy-1-O-methyl-6-α-D-glucopyranosyl)-imino]-D-glucitol as an amorphous solid (0.42 g, 63%).

### EXAMPLE 3

Preparation of 6-O-t-BUTYLDIMETHYLSILYL-2,3,4-TRI-O-BENZYL-1,5-DIDEOXY-[(2,3,4-TRI-O-BENZYL-6-DEOXY-1-O-METHYL-6-α-D-GLUCOPYRANOSYL)IMINO]-D-GLUCITOL

To a suspension of sodium hydride (0.140 g, 5.84 mmol) in dimethyl formamide (20 ml) are added dropwise 6-O-t-butyldimethylsilyl-1,5-dideoxy-1,5-[(6-deoxy-1-O-methyl-6-α-D-glucopyranosyl)imino]-D-glucitol (0.42 g, 0.927 mmol), benzyl bromide (1 g, 5.84 mmol) and n-tetrabutyl ammonium iodide (0.221 g, 0.6 mmol) in dimethyl formamide (30 ml). The mixture is stirred overnight and the resulting mixture is hydrolyzed with aqueous ammonium chloride. Dimethyl formamide is evaporated under high vacuum. The residue is taken with water and extracted three times with ethyl acetate. The organic layer is dried over sodium sulfate, filtered and concentrated under reduced pressure. Flash chromatography on silica gel and elution with a 25:75 mixture of ethyl acetate and hexane affords 6-0-t-butyldimethylsilyl-2,3,4-tri-O-benzyl-1,5-dideoxy-[(2,3,4-tri-O-benzyl-6-deoxy-1-O-methyl-6-α-D-glucopyranosyl)imino]-D-glucitol (0.828 g, 90%) as an amorphous solid.

### EXAMPLE 4

Preparation of 2,3,4-TRI-O-BENZYL-1,5-DIDEOXY-[(2,3,4-TRI-O-BENZYL-6-DEOXY-1-O-METHYL-6-α-D-GLUCOPYRANOSYL)IMINO]-D-GLUCITOL

6-O-t-Butyldimethylsilyl-2,3,4-tri-O-benzyl-1,5-dideoxy-[(2,3,4-tri-O-benzyl-6-deoxy-1-O-methyl-6-a,D-glucopyranosyl)imino]-D-glucitol (0.82 g, 0.825 mmol) is dissolved in tetrahydrofuran (10 ml) n-tetrabutyl ammonium fluoride trihydrate (0.39 g, 1.24 mmol) is added and the mixture is stirred overnight at room temperature. The solvent is evaporated under reduced pressure. The residue is dissolved in ethyl acetate and washed twice with water. The organic layer is dried over sodium sulfate and concentrated under reduced pressure. Flash chromatography on silica gel and elution with a 1:1 mixture of hexane and ethyl acetate affords 2,3,4-tri-O-benzyl-1,5-dideoxy-[(2,3,4-tri-O-benzyl-6-deoxy-1-O-methyl-6-α-D-glucopyranosyl)imino]-D-glucitol as an amorphous solid (0.71 g, 98%).

### EXAMPLE 5

### Preparation of 1,5-DIDEOXY-2,3,4-TRI-O-BENZYL-6-O-(6-O-TRIMETHYLSILYL-2,3,4-TRI-O-BENZYL-6-a-D-GLUCOPYRANOSYL-1,5-[(6-DEOXY-2,3,4-TRI-O-BENZYL-1-O-METHYL-6-α-D-GLUCOPYRANOSYL)-IMINO]-D-GLUCITOL

To a solution of 2,3,4-tri-O-benzyl-6-O-trimethylsilyl-a-D-glucopyranosyl bromide [C. P. Fei and T. H. Chan, Tetrahedron Letters, 28, 849, (1987)] (0.43 g, 0.74 mmol) in ethanol-free chloroform (3 ml) is added diisopropylethylamine (0.5 ml) and 2,3,4-tri-O-benzyl-1,5-dideoxy-[(2,3,4-tri-O-benzyl-6-deoxy-1-O-methyl-6-a-D-glucopyranosyl)-imino]-D-glucitol (0.65 g, 0.74 mmol) in ethanol-free chloroform (3 ml). The mixture is stirred at room temperature during 24 hours. The mixture is diluted with methylene chloride and washed with water. The organic layer is dried over sodium sulfate, filtered and concentrated under reduced pressure to afford an amorphous solid. Flash chromatography on silica gel and elution with a 6:4 mixture of hexane and ethyl acetate affords 1,5-dideoxy-2,3,4-tri-O-benzyl-6-O-(6-O-trimethylsilyl-2,3,4-tri-O-benzyl-6-a-D-glucopyranosyl)-1,5-[(6-deoxy-2,3,4-tri-O-benzyl-1-O-methyl-6-α-D-glucopyranosyl)imino]-D-glucitol as an amorphous white solid (0.307 g, 30%).

### EXAMPLE 6

Preparation of 1,5-DIDEOXY-6-O-(α-D-GLUCOPYRANOSYL)-1,5-[(6-DEOXY-1-O-METHYL-6-α-D-GLUCOPYRANOSYL)IMINO]-D-GLUCITOL

1,5-dideoxy-2,3,4-tri-O-benzyl-6-O-(6-O-trimethylsilyl-2,3,4-tri-O-benzyl-6-a-D-glucopyranosyl)-1,5-[(6-deoxy-2,3,4-tri-O-benzyl-1-O-methyl-6-α-D-glucopyranosyl)imino]-D-glucitol (0.3 g, 0.217 mmol) is dissolved in acetic acid (40 ml). Palladium 10% on charcoal (0.6 g) is added. The mixture is hydrogenated during 7 days at 3 atmosphere. The catalyst is removed by filtration and the solvents are evaporated under reduced pressure. The residue is dissolved in water and passed through a column of Amberlyst A26 OH^{e.} Water is evaporated under reduced pressure and flash chromatography on silica gel and elution with a graded mixture of chloroform, methanol and water affords the expected amine 1,5-dideoxy-6-O-(a-D-glucopyranosyl)-1,5-[(6-deoxy-1-0-methyl-6-a-D-glucopyranosyl)imino]-D-glucitol (0.85 g, 79%) as an amorphous solid.

### EXAMPLE 7

### Preparation of 2,3,6-TRI-O-BENZYL-1,5-DIDEOXY-1,5-IMINO-D-GLUCITOL

To a solution of 2,3,6-tri-O-benzyl-5-deoxy-D-gluconic acid 6-lactam (compound described in Example 75) (0.75 g, 1.6 mmol) in dry tetrahydrofuran (15 ml) was added a 10 M solution of borane in methyl sulfide (0.58 ml) under nitrogen at 0 _{°} C. The mixture was stirred 15 min at 0 ° C, 30 min at room temperature, then refluxed during 6 h and finally stirred overnight at room temperature. The mixture was cooled to 0 _{°} C and the excess of borane was destroyed with methanol and stirred 1 h at room temperature. The reaction mixture was treated with gaseous hydrochloric acid and refluxed during 1 h. The solvents were evaporated under reduced pressure. The residue was dissolved in ethyl acetate and washed with a saturated aqueous solution of sodium bicarbonate. The organic layer was dried over sodium sulfate, filtered and concentrated under reduced pressure to afford an oil. Flash chromacography on silica gel and elution with ethyl acetate afforded 2,3,6-tri-O-benzyl-1,5-dideoxy-1,5-imino-D-glucitol which crystallized in methanol (0.655 g, 90%); m.p. 73-74 ° C.

### EXAMPLE 8

### Preparation of METHYL 2,3,4-TRI-O-BENZYL-6-O-TRIFLUOROMETHYLSULFONYL-a-D-GLUCOPYRANOSIDE

To a solution of dry pyridine (0.46 ml) in methylene chloride (17.5 ml) cooled to -15 _{°} C was added trifluoromethanesulfonic anhydride (0.87 ml). The mixture was stirred during 15 min at -10° C, then methyl 2,3,4-tri-O-benzyl-a-D-glucopyranoside (1.2 g, 2.58 mmol) in methylene chloride (5 ml) was added (P. Kovac, V. Sklenar and C. Glaudemans, Carbohydr. Res., 175, 201 (1988)). The mixture was stirred during 1.5 h at -10° C. The reaction mixture wa̅s̅ w̅a̅s̅h̅e̅d̅ w̅i̅t̅h̅ wa̅t̅e̅r. The organic layer was dried over sodium sulfate, filtered and concentrated under reduced pressure to afford an oil. Flash chromatography on silica gel and elution with a 7:3 mixture of hexane and ethyl acetate afforded the expected compound methyl 2,3,4-tri-O-benzyl-6-0-trifluoromethylsulfonyl-a-D-glucopyranoside which was crystallized from hexane (1.43 g, 93%); m.p. 44-45 ° C.

### EXAMPLE 9

### Preparation of 2,3,6-TRI-O-BENZYL-1,5-DIDEOXY-1,5-[(2,3,4-TRI-O-BENZYL-6-DEOXY-1-O-METHYL-6-α-D-GLUCOPYRANOSYL)IMINO]-D-GLUCITOL

A solution of methyl 2,3,4-tri-O-benzyl-6-0-trifluoromethylsulfonyl-a-D-glucopyranoside (0.7 g, 1.17 mmol) and 2,3,6-tri-O-benzyl-1,5-dideoxy-1,5-imino-D-glucitol (0.509 g, 1.17 mmol) in ethanol-free chloroform (55 ml) was refluxed under nitrogen during 48 h. The mixture was diluted in methylene chloride and washed successively with a saturated aqueous solution of sodium bicarbonate and saturated brine. The organic layer was dried over sodium sulfate, filtered and concentrated under reduced pressure to afford an oil. Flash chromatography on silica gel and elution with a 6:4 mixture of hexane and ethyl acetate afforded the expected compound 2,3,6-tri-O-benzyl-1,5-dideoxy-1,5-[(2,3,4-tri-O-benzyl-6-deoxy-1-O-methyl-6-α-D-glucopyranosyl)imino]-D-glucitol which was crystallized from methanol (0.772 g, 75%); m.p. 102-103 ° C.

### EXAMPLE 10

Preparation of 1,5-DIDEOXY-1,5-[(6-DEOXY-1-O-METHYL-6-α-D-GLUCOPYRANOSYL)IMINO]-D-GLUCITOL

2,3,6-tri-O-benzyl-1,5-dideoxy-1,5-[(2,3,4-tri-O-benzyl-6-deoxy-1-O-methyl-6-α-D-glucopyranosyl)imino]-D-glucitol (0.646 g, 0.73 mmol) was dissolved in methanol (20 ml), cyclohexene (10 ml) and Palladium hydroxide 20% on charcoal (1.2 g) were added. The mixture was degased and refluxed 24 h under argon atmosphere. The catalyst was filtered and washed twice with methanol. The solvents were evaporated under reduced pressure. The residue was dissolved in water, the aqueous phase was extracted twice with ethyl acetate. The aqueous layer was put to dryness under reduced pressure to afford a foam. Flash chromatography on silica gel and elution with a 50:50:4 mixture of methanol, chloroform and water afforded the expected compound 1,5-dideoxy-1,5-[(6-deoxy-1-0-methyl-6-a-D-glucopyranosyl)imino]-D-glucitol as a foam (0.13 g, 52%).

### EXAMPLE 11

### Preparation of METHYL 2,3,4-TRI-O-BENZYL-6,7-DIDEOXY-a-D-GLUCOHEPT-6-ENOPYRANOSIDE

To a solution of oxalyl chloride (1.05 ml, 17.22 mmol) in dry tetrahydrofuran (40 ml) cooled to -78 _{°} C, dry dimethyl sulfoxide (1.3 ml, 18.04 mmol) was added dropwise and then stirred during 35 min at -35 _{°} C. The reaction mixture was cooled again to -78 ° C and methyl 2,3,4-tri-O-benzyl-a-D-glucopyranoside (6 g, 16.4 mmol) dissolved in tetrahydrofuran (20 ml) was added and the mixture was stirred during 15 min at -35 ° C, then triethylamine (11.5 ml, 82.65 mmol) was added and the mixture was stirred during 1 h at -35 _{°} C. This aldehyde was used without purification and isolation in a Wittig reaction described as follows. To dried triphenylmethylphosphonium bromide (11.7 g, 32.8 mmol) suspended in tetrahydrofuran (700 ml) was added dropwise at -78 ° C a 1.42 M solution of n-butyllithium in hexane (23 ml, 32.66 mmol). The reaction mixture was warmed to room temperature and stirred during 1.5 h. Then the mixture was cooled to 0 ° C and potassium tertio-butoxide (3.68 g, 32.8 mmol) and dry tertio-butyl alcohol (3ml, 31.8 mmol) were added. The mixture was stirred again at room temperature during 30 min. The reaction mixture was cooled to -78 ° C and the tetrahydrofuran solution of the aldehyde prepared above was added dropwise. The reaction mixture was warmed to room temperature and stirred during 2 h. A saturated aqueous solution of ammonium chloride was added and the solvents were evaporated under reduced pressure. The residue was dissolved in ether and washed with water. The organic layer was dried over sodium sulfate, filtered and concentrated under reduced pressure to afford a brown oil. Flash chromatography on silica gel and elution with a 4:96 mixture of ethyl acetate and toluene afforded the expected olefin methyl 2,3,4-tri-O-benzyl-6,7- dideoxy-a-D-glucohept-6-enopyranoside (3.26 g, 55%) which crystallized from hexane; m.p. 46-47 ° C.

### EXAMPLE 12

### Preparation of METHYL 2,3,4-TRI-O-BENZYL-6-DEOXY-a-D-GLUCOHEPTOPYRANOSIDE

To a solution of methyl 2,3,4-tri-O-benzyl-6,7-dideoxy-a-D-glucohept-6-enopyranoside (0.878 g, 2.43 mmol) in dry tetrahydrofuran (5 ml) was added a 10 M solution of borane in methyl sulfide (0.24 ml, 2.4 mmol) at 0 ° C under nitrogen. The mixture was stirred during 3 h at room temperature. The excess of borane was destroyed with ethanol (1 ml). The mixture was cooled at 0 _{°} C. 30% hydrogen peroxide (0.3 ml) were added. The mixture was refluxed during 2 h. The reaction mixture was diluted with water and extracted three times with ether. The organic layer was dried over sodium sulfate, filtered and concentrated under reduced pressure to afford an oil. Flash chromatography on silica gel and elution with a 1:1 mixture of ethyl acetate and hexane afforded the expected alcohol methyl 2,3,4-tri-O-benzyl-6-deoxy-a-D-glucohep- topyranoside (0.414 g, 45%) which crystallized from hexane; m.p. 50-53 ° C.

### EXAMPLE 13

### Preparation of METHYL 2,3,4-TRI-O-BENZYL-6-DEOXY-7-O-METHYLSULFONYL-a-D-GLUCOHEP-TOPYRANOSIDE

To a solution of methyl 2,3,4-tri-O-benzyl-6-deoxy-7-0-methylsulfonyl-a-D-glucoheptopyranoside (0.38 g, 0.83 mmol) in dry methylene chloride (10 ml) was added triethylamine (0.2 ml, 1.43 mmol). Then the solution was cooled to -10 ° C and mesylchloride (0.08 ml), 1 mmol) was added. The mixture was stirred an additional 15 min at -10°C, then the reaction was allowed to warm up to room temperature. The mixture was washed three times with water. The organic phase was dried over sodium sulfate, filtered and concentrated under reduced pressure to afford a yellow oil. Flash chromatography on silica gel and elution with a 40:60 mixture of ethyl acetate and hexane afforded the expected mesylate methyl 2,3,4-tri-O-benzyl-6-deoxy-7-0-methylsulfonyl-a-D-glucoheptopyranoside as an oil (0.38 g, 91 %).

### EXAMPLE 14

### Preparation of METHYL 2,3,4-TRI-O-BENZYL-6,7-DIDEOXY-7-IODO-a-D-GLUCOHEPTO-PYRANOSIDE

To a solution of methyl 2,3,4-tri-O-benzyl-6-deoxy-7-0-methylsulfonyl-a-D-glucoheptopyranoside (0.38 g, 0.83 mmol) in ether (5 ml) was added at 0 ° C a 0.375 M solution of magnesium iodide (6.7 ml). The mixture was stirred 15 min at 0 ° C. The excess of magnesium iodide was hydrolyzed with water. The reaction mixture was washed with sodium thiosulfate and water. The organic layer was dried over sodium sulfate, filtered and concentrated under reduced pressure to afford an oil. Flash chromatography on silica gel and elution with a 2:8 mixture of ethyl acetate and hexane afforded the expected iodide methyl 2,3,4-tri-O-benzyl-6,7-dideoxy-7-iodo-α-D-glucoheptopyranoside which was crystallized from hexane (0.368 g, 91%); m.p. 66-68 _{°} C.

### EXAMPLE 15

### Preparation of 2,3,6-TRI-O-BENZYL-1,5-DIDEOXY-1,5-[(2,3,4-TRI-O-BENZYL-6,7-DIDEOXY-1-O-METHYL-7-a-D-GLUCOHEPTOPYRANOSYL)IMINO]-D-GLUCITOL

A solution of methyl 2,3,4-tri-O-benzyl-6,7-dideoxy-7-iodo-a-D-glucoheptopyranoside (0.338 g, 0.69 mmol) and 2,3,6-tri-O-benzyl-1,5-dideoxy-1,5-imino-D-glucitol (0.1 g, 0.23 mmol) in dry dimethylformamide (3 ml) was heated at 80 ° C overnight along with dry potassium carbonate (0.127 g, 0.92 mmol). The dimethylformamide was evaporated under reduced pressure. The residue was taken with ethyl acetate and washed twice with water. The organic layer was dried over sodium sulfate, filtered and concentrated under reduced pressure to afford an oil. Chromatography on neutral aluminum oxide activity III and elution with a 8:2 mixture of hexane and ethyl acetate afforded the expected amine 2,3,6-tri-O-benzyl-1,5-dideoxy-1,5-[-(2,3,4-tri-O-benzyl-6,7-dideoxy-1-O-methyl-7-α-D-glucoheptopyranosyl)imino]-D-glucitol which was crystallized in methanol (0.125 g, 60%); m.p. 42-43 ° C.

### EXAMPLE 16

### Preparation of 1,5-DIDEOXY-1,5-[(6,7-DIDEOXY-1-O-METHYL-7-α-D-GLUCOHEPTOPYRANOSYL)IMINO]-D-GLUCITOL

2,3,6-tri-O-benzyl-1,5-dideoxy-1,5-[(2,3,4-tri-O-benzyl-6,7-dideoxy-1-O-methyl-7-α-D-glucoheptopyranosyl)imino]-D-glucitol (0.1 g, 0.11 mmol) was dissolved in methanol (10 ml) containing ethyl acetate (0.1 ml) and water (1 ml). Palladium hydroxyde 20% on charcoal (0.05 g) was added. The mixture was hydrogenated at 1 atmosphere during two weeks. The catalyst was removed by filtration and the solvents were evaporated under reduced pressure. Crystallization of the residue from isopropanol afforded the expected amine 1,5-dideoxy-1,5-[(6,7-dideoxy-1-O-methyl-7-α-D-glucoheptopyranosyl)imino]-D-glucitol (0.023 g, 58%).

### EXAMPLE 17

### Preparation of 2,3,6-TRI-O-BENZYL-1,5-DIDEOXY-1,5-[(1-DEOXY-2,3:4,5-DI-O-ISOPROPYLlDENE-β-D-FRUCTOPYRANOSYL)IMINO]-D-GLUCITOL

A solution of 2,3:4,5-di-O-isopropylidene-1-0-trifluoromethylsulfonyl-,8-D-fructopyranose (1.20 g, 3.06 mmol) (P.J. Card and W.D. Hitz, J. Amer. Chem. Soc., 106, 5348 (1984)) and 1,5-dideoxy-2,3,6,-tri-O-benzyl-1,5-imino-D-glucitol (1.331 g, 3.06 mmol) in ethanol-free chloroform (70 ml) is refluxed under nitrogen during 60 h. The mixture is diluted with methylene chloride and washed successively with a saturated aqueous bicarbonate solution and saturated brine. The organic layer is dried over sodium sulfate, filtered and concentrated under reduced pressure to afford an oil. Flash chromatography on silica gel and elution with graded mixture of hexane and ethyl acetate will afford the expected amine 2,3,6-tri-O-benzyl-1,5-dideoxy-1,5-[(1-deoxy-2,3:4,5-di-O-isopropylidene-β-D-fructopyranosyl)imino]-D-glucitol as an oil.

### EXAMPLE 18

### Preparation of 2,3,6-TRI-O-BENZYL-1,5-DIDEOXY-1,5-[(1-DEOXY-2-O-METHYL-α-D-FRUC-TOFURANOSYL)IMINO]-D-GLUCITOL

2,3,6-tri-O-benzyl-1,5-dideoxy-1,5-[(1-deoxy-2,3:4,5-di-O-isopropylidene-β-D-fructopyranosyl)imino]-D-glucitol (1.4 g, 2.074 mmol) is dissolved in methanol (100 ml) containing 2% of dry hydrochloric acid. The mixture is refluxed during 48 h. The mixture is neutralized with Amberlyst A 26 OH- form and filtered. The solvents are evaporated under reduced pressure. Flash chromatography on silica gel and elution with graded mixture of ethyl acetate and methanol will afford the expected amine 2,3,6-tri-O-benzyl-1,5-dideoxy-1,5-[(1-deoxy-2-0-methyl-α-D-fructofuranosyl)-imin0]-D-glucitol.

### EXAMPLE 19

### Preparation of 1,5-DIDEOXY-1,5-[(1-DEOXY-2-O-METHYL-α-D-FRUCTOFURANOSYL)IMINO]-D-GLUCITOL

The amine 2,3,6-tri-O-benzyl-1,5-dideoxy-1,5-[(1-deoxy-2-O-methyl-a-D-fructofuranosyl)imino]-D-glucitol (0.617 g, 1.014 mmol) is dissolved in methanol (25 ml) containing water (2.5 ml), palladium hydroxide 20% on charcoal (0.3 g) is added. The mixture is hydrogenated during 4 days at atmospheric pressure. The catalyst is removed by filtration and the solvents are evaporated under reduced pressure. Flash chromatography on silica gel and elution with graded mixture of chloroform, methanol and water will afford the expected amine 1,5-dideoxy-1,5-[1-(deoxy-2-O-methyl-a-D-fructofuranosyl)imino]-D-glucitol as an amorphous solid.

### EXAMPLE 20

### Preparation of METHYL 2,3,6-TRI-O-BENZYL-4-O-TRIFLUOROMETHYLSULFONYL-a-D-GALACTOPYRANOSIDE

To a solution of dry pyridine (0.46 ml) in methylene chloride (17.5 ml) cooled to -15 ° C is added trifluoromethane sulfonic anhydride (0.87 ml). The mixture is stirred during 15 min at -10 ° C, then methyl 2,3,6-tri-O-benzyl-a-D-galacto-pyranoside (1.2 g, 2.58 mmol) in methylene chloride (5 ml) is added (N. Morishima, S. Koto, M. Oshima, A. Sugimoto and S. Zen, Bull. Chem. Soc. Jpn., 56, 2849 (1983)). The mixture is washed with water. The organic layer is dried over sodium sulfate, filtered and concentrated under reduced pressure so as to afford an oil which is the expected triflate methyl 2,3,6-tri-O-benzyl-4-0-trifluoromethyl-sulfonyl-a-D-galactopyranoside.

### EXAMPLE 21

### Preparation of 2,3,6-TRI-O-BENZYL-1,5-DIDEOXY-1,5-[(2,3,6-TRI-O-BENZYL-4-DEOXY-1-O-METHYL-4-α-D-GLUCOPYRANOSYL)IMINO]-D-GLUCITOL

A solution of methyl 2,3,6-tri-O-benzyl-4-0-trifluoromethylsulfonyl-a-D-galactopyranoside (1.25 g, 2.53 mmol) and 2,3,6-tri-O-benzyl-1,5-dideoxy-1,5-imino-D-glucitol (1.098 g, 2.53 mmol) in ethanol-free chloroform (70 ml) is refluxed under nitrogen during 3 days. The mixture is diluted with methylene chloride and washed successively with a saturated aqueous solution of sodium bicarbonate and saturated brine. The organic layer is dried over sodium sulfate, filtered and concentrated under reduced pressure so as to afford an oil. Flash chromatography on silica gel and elution with graded mixture of hexane and ethyl acetate will afford the expected amine 2,3,6-tri-O-benzyl-1,5-dideoxy-1,5-[(2,3,6-tri-O-benzyl-4-deoxy-1-O-methyl-4-a-D-glucopyranosyl)imino]-D-glucitol as an oil.

### EXAMPLE 22

### Preparation of 1,5-DIDEOXY-1,5-[(4-DEOXY-1-O-METHYL-4-α-D-GLUCOPYRANOSYL)IMINO]-D-GLUCITOL

The amine 2,3,6-tri-O-benzyl-1,5-dideoxy-1,5-[(2,3,6-tri-O-benzyl-4-deoxy-1-O-methyl-4-α-D-glucopyranosyl)imino]-D-glucitol (0.911 g, 1.03 mmol) is dissolved in methanol (20 ml). Cyclohexene (10 ml) and palladium hydroxyde 20% on charcoal are added. The mixture is degased and refluxed 16 h under argon atmosphere. The catalyst is filtered and washed twice with methanol. The solvents are evaporated under reduced pressure. The residue is dissolved in water. The aqueous phase is extracted twice with ethyl acetate. The aqueous layer is put to dryness under reduced pressure so as to afford a foam. Flash chromatography on silica gel and elution with a 50:50:4 mixture of methanol, chloroform and water will afford the expected amine 1,5-dideoxy-1,5-[(4-deoxy-1-O-methyl-4-a-D-glucopyranosyl)imino]-D-glucitol as a foam.

### EXAMPLE 23

### Preparation of METHYL 2,3,4-TRI-O-BENZYL-6-O-(2,3,4-TRI-O-BENZYL-6-O-TRIFLUOROMETHYLSULFONYL-a-D-GLUCOPYRANOSYL)-a-D-GLUCOPYRANOSIDE

To a solution of dry pyridine (0.24 ml) in methylene chloride (25 ml) cooled to -15_{°}C is added trifluoromethane sulfonic anhydride (0.45 ml). The mixture is stirred during 15 min at -10 ° C, then methyl 6-O-(2,3,4-tri-O-benzyl-a-D-glucopyranosyl)-2,3,4-tri-O-benzyl-a-D-glucopyranoside 1.2 g, 1.34 mmol) in methylene chloride (5 ml) is added (R. Eby and C. Schuerch, Carbohydr. Res., 50, 203 (1976)). The mixture is stirred during 1.5 h at -10 ° C. The reaction mixture is washe̅d̅ w̅i̅t̅h̅ w̅a̅t̅e̅r̅.̅ T̅he̅ organic layer is dried over sodium sulfate, filtered and concentrated under reduced pressure so as to afford an oil (1.35 g, 98%) which will be the expected triflate methyl-2,3,4-tri-O-benzyl-6-O-(2,3,4-tri-O-benzyl-6-O-trifluoromethylsulfonyl-α-D-glucopyranosyl)-a-D-glucopyranoside.

### EXAMPLE 24

### Preparation of 2,3,6-TRI-O-BENZYL-1,5-DIDEOXY-N-[2,3,4-TRI-O-BENZYL-6-DEOXY-1-(2,3,4-TRI-O-BENZYL-1-O-METHYL-6-O-α-D-GLUCOPYRANOSYL)-α-D-GLUCOPYRANOSYL]-1,5-IMINO-D-GLUCITOL

A solution of methyl 2,3,4-tri-O-benzyl-6-O-(2,3,4-tri-O-benzyl-6-O-trifluoromethysulfonyl-α-D-glucopyranosyl)-a-D-glucopyranoside (1.35 g, 1.31 mmol) and 2,3,6-tri-O-benzyl-1,5-dideoxy-1,5-imino-D-glucitol (0.567 g, 1.31 mmol) in ethanol-free chloroform (50 ml) is refluxed under nitrogen during 48 h. The mixture is diluted with methylene chloride and washed successively with a saturated aqueous solution of sodium bicarbonate and saturated brine. The organic layer is dried over sodium sulfate, filtered and concentrated under reduced pressure so as to afford a foam. Flash chromatography on silica gel and elution with graded mixture of hexane and ethyl acetate will afford the expected amine 2,3,6-tri-O-benzyl-1,5-dideoxy-N-[2,3,4-tri-O-benzyl-6-deoxy-1-(2,3,4-tri-O-benzyl-1-O-methyl-6-O-α-D-glucopyranosyl)-α-D-glucopyranosyl]-1 ,5-imino-D-glucitolas a foam.

### EXAMPLE 25

### Preparation of 1,5-DIDEOXY-N-[6-DEOXY-1-(1-O-METHYL-6-O-α-D-GLUCOPYRANOSYL)-α-D-GLUCOPYRANOSYL]-1,5-IMINO-D-GLUCITOL

The amine 2,3,6-tri-O-benzyl-1,5-dideoxy-N-[2,3,4-tri-O-benzyl-6-deoxy-1-(2,3,4-tri-O-benzyl-1-O-methyl-6-0-a-D-glucopyranosyl)-a-D-glucopyranosyl]-1,5-imino-D-glucitol(l.2 g, 0.915 mmol) is dissolved in methanol (30 ml). Palladium hydroxyde 20% on charcoal (0.5 g) is added. The mixture is hydrogenated during 4 days at 3 atmosphere. The catalyst is removed by filtration and the solvents are evaporated under reduced pressure. Flash chromatography on silica gel and elution with graded mixture of chloroform, methanol and water will afford the expected amine 1,5-dideoxy-N-[6-deoxy-1-(1-O-methyl-6-O-α-D-glucopyranosyl)-α-D-glucopyranosyl]-1,5-imino-D-glucitol as a foam.

### EXAMPLE 26

### Preparation of METHYL 6-O-(2,3,4-TRI-O-BENZYL-6,7-DIDEOXY-a-D-GLUCOHEPT-6-ENOPYRANOSYL)-2,3,4-TRI-O-BENZYL-a-D-GLUCOPYRANOSIDE

To a solution of oxalyl chloride (0.37 ml, 5.97 mmol) in dry tetrahydrofuran (40 ml) cooled to -78 ° C, dry dimethyl sulfoxide (0.45 ml, 6.26 mmol) is added dropwise and then stirred during 35 min at -35 ° C. The reaction mixture is cooled again to -78 ° C and methyl 6-O-(2,3,4-tri-O-benzyl-a-D-glucopyranosyl)-2,3,4-tri-O-benzyl-a-D-glucopyranoside (5.1 g, 5.69 mmol) dissolved in tetrahydrofuran (20 ml) is added and the mixture is stirred during 15 min at -35 ° C, then triethylamine (3.96 ml, 28.45 mmol) is added and the mixture is stirred during 1 h at - 35 _{°} C. This aldehyde is to be used without purification and isolation in a Wittig reaction described as follows. To dried triphenylmethylphosphonium bromide (4.059 g, 11.38 mmol) suspended in tetrahydrofuran (100 ml) is added dropwise at -78 ° C a 1.55 M solution of n-butyllithium in hexane (7.34 ml, 11.38 mmol). The reaction mixture is warmed to room temperature and stirred during 1.5 h. Then the mixture is cooled to 0 ° C and potassium tertio-butoxide (1.275 g, 11.38 mmol) and dry tertio-butyl alcohol (1.04 ml, 11.38 mmol) are added. The mixture is stirred again at room temperature during 30 min. The reaction mixture is cooled to -78 ° C and the tetrahydrofuran solution of the aldehyde prepared above is added dropwise. The reaction mixture is warmed to room temperature and stirred during 2 h. A saturated aqueous solution of ammonium chloride is added and the solvents are evaporated under reduced pressure. The residue is dissolved in ethyl acetate and washed with water. The organic layer is dried over sodium sulfate, filtered and concentrated under reduced pressure so as to afford a brown oil. Flash chromatography on silica gel and elution with a graded mixture of carbon tetrachloride and ethyl acetate will afford the expected olefin methyl 6-O-(2,3,4-tri-O-benzyl-6,7-dideoxy-a-D-glucohept-6-enopyranosyl)-2,3,4-tri-O-benzyl-a-D-glucopyranoside as an amorphous solid.

### EXAMPLE 27

### Preparation of METHYL 6-O-(2,3,4-TRI-O-BENZYL-6-DEOXY-a-D-GLUCOHEPTOPYRANOSYL)-2,3,4-TRI-O-BENZYL-a-D-GLUCOPYRANOSIDE

To a solution of methyl 6-O-(2,3,4-tri-O-benzyl-6,7-dideoxy-α-D-glucohept-6-enopyranosyl)-2,3,4-tri-O-benzyl-a-D-glucopyranoside (2.54 g, 2.85 mmol) in dry tetrahydrofuran (10 ml) is added a 10 M solution of borane in methyl sulfide (0.28 ml, 2.8 mmol) at 0 ° C under nitrogen. The mixture is stirred during 3 h at room temperature. Then the mixture is cooled to 0 ° C. The excess of borane is destroyed with ethanol (1 ml). The mixture is cooled at 0 ° C. 30% hydrogen peroxide (0.3 ml) and 3 N aqueous solution of sodium hydroxyde (0.3 ml) are added. The mixture is refluxed during 2 h. The reaction mixture is diluted with water and extracted three times with ethyl acetate. The organic layer is dried over sodium sulfate, filtered and concentrated under reduced pressure so as to afford a foam. Flash chromatography on silica gel and elution with a graded mixture of carbon tetrachloride and ethyl acetate will afford the expected alcohol methyl 6-O-(2,3,4-tri-O-benzyl-6-deoxy-a-D-glucoheptopyranosyl)-2,3,4-tri-O-benzyl-a-D-glucopyranoside as a foam.

### EXAMPLE 28

### Preparation of METHYL 6-O-(2,3,4-TRI-O-BENZYL-6,7-DIDEOXY-7-IODO-a-D-GLUCO-HEP-TOPYRANOSYL)-2,3,4-TRI-O-BENZYL-a-D-GLUCOPYRANOSIDE

To a solution of methyl 6-O-(2,3,4-tri-O-benzyl-6-deoxy-a-D-glucoheptopyranosyl)-2,3,4-tri-O-benzyl-a-D-glucopyranoside (1.245 g, 1.37 mmol) in dry methylene chloride (15 ml) is added triethylamine (0.29 ml, 2.05 mmol). Then the solution is cooled to -10_{°}C, and mesylchloride (0.11 ml, 1.42 mmol) is added dropwise. The mixture is stirred an additional 15 min at -10 ° C, then the reaction mixture is washed three times with water. The organic layer is dried over sodium sulfate, filtered and concentrated under reduced pressure so as to afford a foam which is to be used without further purification. The crude methyl 6-O-(2,3,4-tri-O-benzyl-6-deoxy-7-O-methylsulfonyl-a-D-glucoheptopyranosyl)-2,3,4-tri-O-benzyl-a-D-glucopyranoside is dissolved in ether (20 ml). To this mixture a 0.35 M solution of magnesium iodide in ether (17.5 ml) is added dropwise at 0 ° C. The excess of magnesium iodide is hydrolyzed with water. The reaction mixture is washed with sodium thiosulfate and water. The organic layer is dried over sodium sulfate, filtered and concentrated under reduced pressure to afford a foam. Flash chromatography on silica gel and elution with a graded mixture of carbon tetrachloride and ethyl acetate will afford the expected iodide methyl 6-O-(2,3,4-tri-O-benzyl-6,7-dideoxy-7-iodo-a-D-glucoheptopyranosyl)-2,3,4-tri-O-benzyl-a-D-glucopyranoside as a foam.

### EXAMPLE 29

### Preparation of 2,3,6-TRI-O-BENZYL-1,5-DIDEOXY-N-[2,3,4-TRI-O-BENZYL-6,7-DIDEOXY-1-(2,3,4-TRI-O-BENZYL-1-O-METHYL-6-O-α-D-GLUCOPYRANOSYL)-7-α-D-GLUCOHEPTOPYRANOSYL]-1,5-IMINO-D-GLUCITOL

A solution of the iodide methyl 6-O-(2,3,4-tri-O-benzyl-6,7-dideoxy-7-iodo-a-D-glucoheptopyranosyl)-2,3,4-tri-O-benzyl-a-D-glucopyranoside (1.145 g, 1.122 mmol) and the amine 2,3,6-tri-O-benzyl-1,5-dideoxy-1,5-imino-D-glucitol (0.162 g, 0.374 mmol) in dry dimethylformamide (4 ml) is heated at 80 ° C overnight along with dry potassium carbonate (0.206 g, 1.49 mmol). The dimethylformamide is evaporated under reduced pressure. The residue is taken with ethyl acetate and washed twice with water. The organic layer is dried over sodium sulfate, filtered and concentrated under reduced pressure so as to afford a foam. Chromatography on neutral aluminum oxide activity III and elution with a graded mixture of carbon tetrachloride and ethyl acetate will afford the expected amine 2,3,6-tri-0-benzyl-1,5-dideoxy-N-[2,3,4-tri-0-benzyl-6,7-dideoxy-1-(2,3,4-tri-O-benzyl-1-O-methyl-6-O-α-D-glucopyranosyl)-7-α-D-glucoheptopyranosyl]-1,5-imino-D-glucitol as a foam.

### EXAMPLE 30

### Preparation of 1,5-DIDEOXY-N-[6,7-DIDEOXY-1-(1-O-METHYL-6-O-α-D-GLUCOPYRANOSYL)-7-α-D-GLUCOHEPTOPYRANOSYL]-1,5-IMINO-D-GLUCITOL

The amine 2,3,6-tri-O-benzyl-1,5-dideoxy-N-[2,3,4-tri-O-benzyl-6,7-dideoxy-1-(2,3,4-tri-O-benzyl-1-O-methyl-6-O-a-D-glucopyranosyl)-7-a-D-glucoheptopyranosyl]-1,5-imino-D-glucitol (0.337 g, 0.254 mmol) is dissolved in methanol (30 ml). Palladium hydroxyde 20% on charcoal (0.4 g) is added. The mixture is hydrogenated during 4 days at 3 atmospheres. The catalyst is removed by filtration and the solvents are evaporated under reduced pressure. Flash chromatography on silica gel and elution with a graded mixture of chloroform, methanol and water will afford the expected amine 1,5-dideoxy-N-[6,7-dideoxy-1-(1-O-methyl-6-O-α-D-glucopyranosyl)-7-α-D-glucoheptopyranosyl]-1,5-imino-D-glucitol as an amorphous solid.

### EXAMPLE 31

### Preparation of METHYL 2,3,6-TRI-0-BENZYL-4-CYANO-4-DEOXY-a-D-GLUCOPYRANOSIDE

A solution of methyl 2,3,6-tri-O-benzyl-4-O-trifluoromethylsulfonyl-a-D-galactopyranoside (3 g, 6.07 mmol) and tetra-n-butyl ammonium cyanide (6.51 g, 24.28 mmol) in ethanol-free chloroform (60 ml) is refluxed under nitrogen during 24 h. The reaction mixture is diluted with methylene chloride, washed twice with water. The organic layer is dried over sodium sulfate, filtered and concentrated under reduced pressure so as to afford an oil. Flash chromatography on silica gel and elution with a graded mixture of hexane and ethyl acetate will afford the expected nitrile methyl 2,3,6-tri-O-benzyl-4-cyano-4-deoxy-a-D-glucopyranoside as an oil.

### EXAMPLE 32

### Preparation of METHYL 2,3,6-TRI-0-BENZYL-4-DEOXY-4-FORMYL-a-D-GLUCOPYRANOSIDE

To a solution of methyl 2,3,6-tri-O-benzyl-4-cyano-4-deoxy-a-D-glucopyranoside (1.75 g, 3.7 mmol) in dry tetrahydrofuran (10 ml) is added dropwise at -78 ° C a 1.2 M solution of diisobutyl aluminum hydride in n-hexane (3.1 ml). The mixture is stirred under argon at -78 °C during 3 h. Methanol (2 ml) is added and the mixture is warmed to 0 ° C. Then the solvents are evaporated under reduced pressure. Ether (50 ml) and 0.1 N aqueous hydrochloric acid (40 ml) are added, the mixture is stirred at 0 ° C during 1 h. Then after decantation the organic layer is dried over sodium sulfate, filtered and concentrated under reduced pressure so as to afford the expected aldehyde methyl 2,3,6-tri-O-benzyl-4-deoxy-4-formyl-a-D-glucopyranoside as an oil which is used without purification.

### EXAMPLE 33

### Preparation of METHYL 2,3,6-TRI-O-BENZYL-4-DEOXY-4-HYDROXYMETHYL-a-D-GLUCO-PYRANOSIDE

The aldehyde methyl 2,3,6-tri-O-benzyl-4-deoxy-4-formyl-a-D-glucopyranoside (1.7 g, 3.57 mmol) is dissolved in ethanol (15 ml). The mixture is cooled to 0 ° C and solid sodium borohydride (0.068 g, 1.8 mmol) is added portionwise. The mixture is stirred 1 h at 0 _{°} C. Then acetic acid (0.4 ml) is added and the solvents are evaporated under reduced pressure. The residue is taken up with ethyl acetate and washed with saturated aqueous sodium bicarbonate and saturated brine. The organic layer is dried over sodium sulfate, filtered and concentrated under reduced pressure so as to afford an oil. Flash chromatography over silica gel and elution with a graded mixture of hexane and ethyl acetate will afford the expected alcohol methyl 2,3,6-tri-O-benzyl-4-deoxy-4-hydroxymethyl-a-D-glucopyranoside as an oil.

### EXAMPLE 34

### Preparation of METHYL 2,3,6-TRI-O-BENZYL-4-DEOXY-4-TRIFLUOROMETHYLSULFONYLOXYMETHYL-a-D-GLUCOPYRANOSIDE

To a solution of dry pyridine (0.45 ml) in methylene chloride (30 ml) cooled to -15_{°}C is added trifluoromethanesulfonic anhydride (0.84 ml). The mixture is stirred during 15 min at -10 ° C, then methyl 2,3,6-tri-O-benzyl-4-deoxy-4-hydroxymethyl-a-D-glucopyranoside (1.19 g, 2.49 mmol) in methylene chloride (5 ml) is added. The mixture is stirred during 1.5 h at -10_{°}C. The reaction mixture is washed with water. The organic layer is dried over sodium sulfate, filtered and concentrated under reduced pressure so as to afford an oil which is the expected triflate methyl 2,3,6-tri-O-benzyl-4-deoxy-4-trifluoromethylsulfonylox- ymethyl-a-D-glucopyranoside.

### EXAMPLE 35

### Preparation of 2,3,6-TRI-O-BENZYL-1,5-DIDEOXY-1,5-[(2,3,6-TRI-O-BENZYL-4-DEOXY-1-O-METHYL-4-α-D-GLUCOPYRANOSYL)METHYLIMINO]-D-GLUCITOL

A solution of methyl 2,3,6-tri-O-benzyl-4-deoxy-4-trifluoromethylsulfonyloxymethyl-a-D-glucopyranoside (1 g, 1.64 mmol) and 2,3,6-tri-O-benzyl-1,5-dideoxy-1,5-imino-D-glucitol (0.71 g, 1.64 mmol) in ethanol-free chloroform (60 ml) is refluxed under nitrogen during 48 h. The mixture is diluted in methylene chloride and washed successively with a saturated aqueous solution of sodium bicarbonate and saturated brine. The organic layer is dried over sodium sulfate, filtered and concentrated under reduced pressure so as to afford a foam. Flash chromatography on silica gel and elution with a graded mixture of hexane and ethyl acetate will afford the expected amine 2,3,6-tri-O-benzyl-1,5-dideoxy-1,5-[(2,3,6-tri-O-benzyl-4-deoxy-1-O-methyl-4-a-D-glucopyranosyl)-methylimino]-D-glucitol as a foam.

### EXAMPLE 36

### Preparation of 1,5-DIDEOXY-1,5-[(4-DEOXY-1-O-METHYL-4-α-D-GLUCOPYRANOSYL)METHYLIMINO]-D-GLUCITOL

The amine 2,3,6-tri-O-benzyl-1,5-dideoxy-1,5-[(2,3,6-tri-O-benzyl-4-deoxy-1-O-methyl-4-α-D-glucopyranosyl)methylimino]-D-glucitol (0.98 g, 1.09 mmol) is dissolved in methanol (20 ml). Cyclohexene (10 ml) and palladium hydroxyde 20% on charcoal (0.8 g) are added and the mixture is refluxed under nitrogen during 8 h. The catalyst is removed by filtration and the solvents are evaporated under reduced pressure. Flash chromatography on silica gel and elution with a graded mixture of chloroform, methanol and water will afford the expected amine 1,5-dideoxy-1,5-[(4-deoxy-I-O-methyl-4-a-D-glucopyranosyl)-methylimino]-D-glucitol as an amorphous solid.

### EXAMPLE 37

### Preparation of 2,3,6-TRI-O-BENZYL-D-GALACTOPYRANOSE

Methyl 2,3,6-tri-O-benzyl-a-D-galactopyranoside (5 g, 10.775 mmol) is dissolved at 0 _{°} C in a 9:1 mixture of trifluoroacetic acid and water (50 ml) [N. Morishima, S. Koto, M. Oshima, A. Sugimoto and S. Zen, Bull. Chem. Soc. Jpn., 56, 2849 (1983)]. The mixture is stirred overnight at 0 ° C. The solvents are evapora̅t̅e̅d̅ under reduced pressure without heating. The residue is dissolved in ethyl acetate and washed successively with sodium bicarbonate and brine. The organic layer is dried over sodium sulfate, filtered and concentrated under reduced pressure so as to afford an oil. Flash chromatography on silica gel and elution with a graded mixture of ethyl acetate and hexane will afford 2,3,6-tri-O-benzyl-D-galactopyranose as an oil.

### EXAMPLE 38

### Preparation of 1,4-DI-O-ACETYL-2,3,6-TRI-O-BENZYL-D-GALACTOPYRANOSE

2,3,6-tri-O-benzyl-D-galactopyranose (3.927 g, 8.72 mmol) is dissolved in dry pyridine (25 ml) and acetic anhydride (5 ml) is added. The mixture is stirred during 24 h at room temperature. The solvent is evaporated under high vacuum. The residue is dissolved in ethyl acetate and washed with water. The organic layer is dried over sodium sulfate, filtered and concentrated under reduced pressure so as to afford the expected diacetate 1,4-di-O-acetyl-2,3,6-tri-O-benzyl-D-galactopyranose (4.64 g, 99%) as an oil which can be used without purification.

### EXAMPLE 39

### Preparation of 4-O-ACETYL-2,3,6-TRI-O-BENZYL-α-D-GALACTOPYRANOSYL CHLORIDE

A solution of 1,4-di-O-acetyl-2,3,6-tri-O-benzyl-D-galactopyranose (4.64 g, 8.67 mmol) in ether (10 ml) is treated with ethereal hydrogen chloride (0.2 g/ml, 25 ml). The mixture is stirred at room temperature during 48 h. The solvents are evaporated under reduced pressure so as to afford an oil. Flash chromatography on silica gel and elution with a graded mixture of carbon tetrachloride and ethyl acetate will afford 4-O-acetyl-2,3,6-tri-O-benzyl-a-D-galactopyranosyl chloride as an oil.

### EXAMPLE 40

### Preparation of METHYL 4-O-(4-O-ACETYL-2,3,6-TRI-O-BENZYL-α-D-GALACTOPYRANOSYL)-2,3,6-TRI-O-BENZYL-a-D-GLUCOPYRANOSIDE

Ethereal silver perchlorate (0.08 M, 84.5 ml, 6.76 mmol) is added with stirring at -30 ° C to a solution of methyl-2,3,6-tri-O-benzyl-a-D-glucopyranoside (2.284 g, 4.93 mmol) (P.J. Garegg, H. Hultberg and S. Wallin, Carbohydr. Res., 108, 97 (1982)), 4-O-acetyl-2,3,6-tri-O-benzyl-a-D-galactopyranosyl chloride (3.142 g, 6.154 mmol) and 2,4,6-trimethylpyridine (0.89 ml, 6.76 mmol) in ether (20 ml). The mixture is stirred 15 min at -30 °C and silver chloride precipitated. The mixture is filtered through a celite pad, the solids are washed with ether, the filtrate is concentrated under reduced pressure. The residue is dissolved in methylene chloride and the organic layer is washed successively with aqueous sodium thiosulfate and water. The organic layer is dried over sodium sulfate, filtered and concentrated under reduced pressure so as to afford a foam. Flash chromatography on silica gel and elution with a graded mixture of hexane and ethyl acetate will afford methyl 4-O-(4-O-acetyl-2,3,6-tri-O-benzyl-α-D-galactopyranosyl)-2,3,6-tri-O-benzyl-α-D-glucopyranosideas a foam.

### EXAMPLE 41

### Preparation of METHYL 2,3,6-TRI-O-BENZYL-4-O-(2,3,6-TRI-O-BENZYL-α-D-GALACTOPYRANOSYL)-α-D-GLUCOPYRANOSIDE

Methyl 4-O-(4-O-acetyl-2,3,6-tri-O-benzyl-α-D-galactopyranosyl)-2,3,6-tri-O-benzyl-α-D-glucopyranoside (2.543 g, 2.71 mmol) is dissolved in hot toluene (20 ml) and methanol (80 ml) is added, followed by a few drops of 1 M methanolic sodium methoxide. The mixture is stirred at room temperature during 2 h. The reaction mixture is made neutral with Amberlite IR 120 (H⁺) resin, filtered and concentrated under reduced pressure so as to afford methyl 2,3,6-tri-O-benzyl-4-O-(2,3,6-tri-O-benzyl-a-D-galactopyranosyl)-a-D-glucopyranoside as an amorphous solid.

### EXAMPLE 42

### Preparation of METHYL 4-O-(2,3,6-TRI-O-BENZYL-4-O-TRIFLUOROMETHYLSULFONYL-α-D-GALACTOPYRANOSYL)-2,3,6-TRI-O-BENZYL-a-D-GLUCOPYRANOSIDE

To a solution of dry pyridine (0.49 ml) in dry methylene chloride (40 ml) cooled to -15 ° C is added trifluoromethane sulfonic anhydride (0.91 ml). The mixture is stirred during 15 min at -10 ° C, then methyl 2,3,6-tri-O-benzyl-4-O-(2,3,6-tri-O-benzyl-a-D-galactopyranosyl)-a-D-glucopyranoside (2.428 g, 2.71 mmol) in methylene chloride (10 ml) is added. The mixture is stirred during 1.5 h at -10 ° C. The reaction mixture is washed with water. The organic layer is dried over sodium sulfate, filtered and concentrated under reduced pressure so as to afford an oil which will be the expected triflate methyl 4-O-(2,3,6-tri-O-benzyl-4-O-trifluoromethylsulfonyl-a-D-galactopyranosyl)-2,3,6-tri-O-benzyl-a-D-glucopyranoside.

### EXAMPLE 43

### Preparation of 2,3,6-TRI-O-BENZYL-1,5-DIDEOXY-N-[2,3,6-TRI-O-BENZYL-4-DEOXY-1-(2,3,6-TRI-O-BENZYL-1-O-METHYL-4-O-α-D-GLUCOPYRANOSYL)-α-D-GLUCOPYRANOSYL]-1,5-IMINO-D-GLUCITOL

A solution of methyl 4-O-(2,3,6-tri-O-benzyl-4-O-trifluoromethysulfonyl-α-D-galactopyranosyl)-2,3,6-tri-O-benzyl-a-D-glucopyranoside (1.52 g, 1.46 mmol) and 2,3,6-tri-O-benzyl-1,5-dideoxy-1,5-imino-D-glucitol (0.632 g, 1.46 mmol) in ethanol-free chloroform (50 ml) is refluxed under nitrogen during 48 h. The mixture is diluted in methylene chloride and washed successively with a saturated aqueous solution of sodium bicarbonate and saturated brine. The organic layer is dried over sodium sulfate, filtered and concentrated under reduced pressure so as to afford a foam. Flash chromatography on silica gel and elution with a graded mixture of hexane and ethyl acetate will afford the expected amine 2,3,6-tri-O-benzyl-1,5-dideoxy-N-[2,3,6-tri-O-benzyl-4-deoxy-1-(2,3,6-tri-O-benzyl-1-O-methyl-4-O-α-D-gluco-pyranosyl)-α-D-glucopyranosyl]-1,5-imino-D-glucitol as an amorphous solid.

### EXAMPLE 44

### Preparation of 1,5-DIDEOXY-N-[4-DEOXY-1-(1-O-METHYL-4-O-α-D-GLUCOPYRANOSYL)-α-D-GLUCOPYRANOSYL]-1,5-IMINO-D-GLUCITOL

2,3,6-tri-O-benzyl-1,5-dideoxy-N-[2,3,6-tri-O-benzyl-4-deoxy-1-(2,3,6-tri-O-benzyl-1-O-methyl-4-O-α-D-glucopyranosyl)-a-D-glucopyranosyl]-1,5-imino-D-glucitol (1 g, 0.762 mmol) is dissolved in methanol (30 ml). Palladium hydroxyde 20% on charcoal (0.5 g) is added. The mixture is hydrogenated during 4 days at 3 atmospheres. The catalyst is removed by filtration and the solvents are evaporated under reduced pressure. Flash chromatography on silica gel and elution with a graded mixture of chloroform, methanol and water will afford the expected amine 1,5-dideoxy-N-[4-deoxy-l-(1-0-methyl-4-0-a-D-glucopyranosyl)-a-D-glucopyranosyl]-1,5-imino-D-glucitol as an amorphous solid.

### EXAMPLE 45

### Preparation of 1-ETHENYL-1,2:3,4-DI-O-ISOPROPYLIDENE-β-D-ARABINOPYRANOSE

To a solution of oxalyl chloride (1.05 ml, 17.22 mmol) in dry tetrahydrofuran (40 ml) cooled to -78 _{°} C, dry dimethyl sulfoxide (1.3 ml, 18.04 mmol) is added dropwise and then stirred during 35 min at -35 _{°} C. The reaction mixture is cooled again to -78 ° C and 2,3:4,5-di-O-isopropylidene-D-fructopyranose (4.26 g, 16.4 mmol) (R.F. Brady, Carbohydr. Res., 15, 35 (1970)) dissolved in tetrahydrofuran (20 ml) is added and the mixture is stirred during 15 min at -35 °C, then triethylamine (11.5 ml, 82.65 mmol) is added and the mixture is stirred during 1 h at -35 °C. This aldehyde can be used without purification and isolation in a wittig reaction described as follows. To dried triphenylmethylphosphonium bromide (11.7 g, 32.8 mmol) suspended in tetrahydrofuran (400 ml) is added dropwise at -78 ° C a 1.55 M solution of n-butyllithium in hexane (21 ml, 32.66 mmol). The reaction mixture is warmed to room temperature and stirred during 1.5 h. Then the mixture is cooled to 0 ° C and potassium tertio-butoxide (3.68 g, 32.8 mmol) and dry tertio-butyl alcohol (3 ml, 31.8 mmol) are added. The mixture is stirred again at room temperature during 30 min. The reaction mixture is cooled to -78 ° C and the tetrahydrofuran solution of the aldehyde prepared above is added dropwise. The reaction mixture is warmed to room temperature and stirred during 2 h. A saturated aqueous solution of ammonium chloride is added and the solvents are evaporated under reduced pressure. The residue is dissolved in ether and washed with water. The organic layer is dried over sodium sulfate, filtered and concentrated under reduced pressure so as to afford a brown oil. Flash chromatography on silica gel and elution with a graded mixture of hexane and ethyl acetate will afford the expected olefin 1- ethenyl-1,2:3,4-di-O-isopropylidene-β-D-arabinopyranose as an oil.

### EXAMPLE 46

### Preparation of 1,2:3,4-DI-O-ISOPROPYLlDENE-1-(2-HYDROXYETHYL)-β-D-ARABINOPYRANOSE

To a solution of 1-ethenyl-1,2:3,4-di-O-isopropylidene-β-D-arabinopyranose (2 g, 7.81 mmol) in dry tetrahydrofuran (15 ml) is added a 10 M solution of borane in methyl sulfide (0.78 ml, 7.8 mmol) at 0 ° C under nitrogen. The mixture is stirred during 3 h at room temperature. The excess of borane is destroyed with ethanol (3 ml). The mixture is cooled at 0 _{°} C. 30% hydrogen peroxide (1 ml) and 3 N aqueous solution of sodium hydroxyde (1 ml) are added. The mixture is refluxed during 2 h. The reaction mixture is diluted with water and extracted three times with ethyl acetate. The organic layer is dried over sodium sulfate, filtered and concentrated under reduced pressure so as to afford an oil. Flash chromatography on silica gel and elution with a 1:1 mixture of ethyl acetate and hexane will afford the expected alcohol 1,2:3,4-di-0-isopropylidene-1-(2-hydroxyethyl)-β-D-arabinopyranose as an oil.

### EXAMPLE 47

### Preparation of 1,2:3,4-DI-O-ISOPROPYLIDENE-1-(2-IODOETHYL)-β-D-ARABINOPYRANOSE

To a solution of 1,2:3,4-di-O-isopropylidene-1-(2-hydroxyethyl)-β-D-arabinose(1.7 g, 6.2 mmol) in dry methylene chloride (30 ml) is added triethylamine (1.3 ml, 9.3 mmol). Then the mixture is cooled to -10_{°}C and mesylchloride (0.5 ml, 6.46 mmol) is added dropwise. The mixture is stirred an additional 15 min at -10 ° C, then the reaction is allowed to warm up to room temperature. The mixture is washed three times with water. The organic phase is dried over sodium sulfate, filtered and concentrated under reduced pressure so as to afford a yellow oil which can be used without purification. The crude 1,2:3,4-di-O-isopropylidene-1-(2-methylsulfonyloxyethyl)-β-D-arabinose is dissolved in ether (15 ml). To this mixture a 0.35 M solution of magnesium iodide in ether (53 ml) is added at 0 ° C. The mixture is stirred 15 min at 0 ° C. The excess of magnesium iodide is hydrolyzed with water. The reaction mixture is washed with aqueous sodium thiosulfate and water. The organic layer is dried over sodium sulfate, filtered and concentrated under reduced pressure so as to afford an oil. Flash chromatography on silica gel and elution with a 9:1 mixture of hexane and ethyl acetate will afford the expected iodide 1,2:3,4-di-0-isopropylidene-1-(2-iodoethyl)-β-D-arabinopyranose as a slightly yellow oil.

### EXAMPLE 48

### Preparation of 2,3,6-TRI-O-BENZYL-1,5-DIDEOXY-1,5-{[2-(1,2:3,4-DI-O-ISOPROPYLIDENE-1-β-D-AR-ABINOPYRANOSYL)ETHYL]IMINOI-D-GLUCITOL

A solution of 1,2:3,4-di-O-isopropylidene-1-(2-iodoethyl)-β-D-arabinose (1.9 g, 4.95 mmol) and 2,3,6-tri-0-benzyl-1,5-dideoxy-1,5-imino-D-glucitol (0.714 g, 1.65 mmol) in dry dimethylformamide (10 ml) is heated at 80 ° C overnight along with dry potassium carbonate (0.91 g, 6.6 mmol). The dimethylformamide is evaporated under reduced pressure. The residue is taken with ethyl acetate and washed twice with water. The organic layer is dried over sodium sulfate, filtered and concentrated under reduced pressure so as to afford an oil. Chromatography on neutral aluminum oxide activity III and elution with a graded mixture of hexane and ethyl acetate will afford the expected amine 2,3,6-tri-O-benzyl-1,5-dideoxy-1,5{[2-(1,2:3,4-di-O-isopropylidene-1-β-D-arabinopyranosyl)ethyl]imino}-D-glucitol as a foam.

### EXAMPLE 49

### Preparation of 2,3,6-TRI-O-BENZYL-1,5-DIDEOXY-1,5-{[2-(1-O-METHYL-1-α-D-ARABINOFURANOSYL)-ETHYL]IMINOI-D-GLUCITOL

2,3,6-tri-O-benzyl-1,5-dideoxy-1,5-{[2-(1,2:3,4-di-O-isopropylidene-1-β-D-arabinopyranosyl)ethyl]imino}-D-glucitol (0.739 g, 1.072 mmol) is dissolved in methanol (60 ml) containing 5% of dry hydrochloric acid and is refluxed during 24 h. The reaction mixture is cooled to room temperature and neutralized with Amberlyst A26 OH- form. The mixture is filtered and the solvent is evaporated under reduced pressure so as to give a foam. Flash chromatography on silica gel and elution with a graded mixture of ethyl acetate and methanol will afford the expected amine 2,3,6-tri-O-benzyl-1,5-dideoxy-1,5-{[2-(1-O-methyl-1-α-D-ar- abinofuranosyl)ethyl]-iminol-D-glucitol as a foam.

### EXAMPLE 50

### Preparation of 1,5-DIDEOXY-1,5-{[2-(1-O-METHYL-1-α-D-ARABINOFURANOSYL)ETHYL]-IMINO}-D-GLUCITOL

The amine 2,3,6-tri-O-benzyl-1,5-dideoxy-1,5-{[2-(1-O-methyl-1-α-D-arabinofuranosyl)ethyl]imino}-D-glucitol (0.4 g, 0.642 mmol) is dissolved in a 9:1 mixture of methanol and water (20 ml). Palladium hydroxyde 20% on charcoal (0.2 g) is added and the mixture is hydrogenated during 4 days at atmospheric pressure. The catalyst is removed by filtration and the solvents are evaporated under reduced pressure. Flash chromatography on silica gel and elution with a graded mixture of chloroform, methanol and water will afford the expected amine 1,5-dideoxy-1,5-{[2-(1-O-methyl-1-α-D-arabinofuranosyl)ethyl]-imino}-D-glucitol as an amorphous solid.

### EXAMPLE 51

### Preparation of METHYL 6-O-(4-O-ACETYL-2,3,6-TRI-O-BENZYL-α-D-GALACTOPYRANOSYL)-2,3,4-TRI-O-BENZYL-a-D-GLUCOPYRANOSIDE

Ethereal silver perchlorate (0.08 M, 76.9 ml, 6.15 mmol) is added with stirring at -30 ° C to a solution of methyl 2,3,4-tri-O-benzyl-a-D-glucopyranoside (2.078 g, 4.48 mmol), 4-O-acetyl-2,3,6-tri-O-benzyl-a-D-galactopyranosyl chloride (2.859 g, 5.6 mmol) and 2,4,6-trimethylpyridine (0.81 ml, 6.15 mmol) in ether (20 ml). The mixture is stirred 15 min at -30 ° C and silver chloride precipitated. The mixture is filtered through a celite pad, the solids are washed with ether, the filtrate is concentrated under reduced pressure. The residue is dissolved in methylene chloride and the organic layer is washed successively with aqueous sodium thiosulfate and water. The organic layer is dried over sodium sulfate, filtered and concentrated under reduced pressure so as to afford a foam. Flash chromatography on silica gel and elution with a graded mixture of hexane and ethyl acetate will afford methyl 6-O-(4-O-acetyl-2,3,6-tri-O-benzyl-α-D-galactopyranosyl)-2,3,4-tri-O-benzyl-a-D-glucopyranosideas a foam.

### EXAMPLE 52

### Preparation of METHYL 2,3,4-TRI-O-BENZYL-6-O-(2,3,6-TRI-O-BENZYL-α-D-GALACTOPYRANOSYL)-α-D-GLUCOPYRANOSIDE

Methyl 6-O-(4-O-acetyl-2,3,6-tri-O-benzyl-α-D-galactopyranosyl)-2,3,4-tri-O-benzyl-α-D-glucopyranoside (2.314 g, 2.46 mmol) is dissolved in hot toluene (20 ml) and methanol (80 ml) is added, followed by a few drops of 1 M methanolic sodium methoxide. The mixture is stirred at room temperature during 2 h. The reaction mixture is made neutral with Amberlite IR 120 (H⁺) resin, filtered and concentrated under reduced pressure so as to afford methyl 2,3,4-tri-O-benzyl-6-O-(2,3,6-tri-O-benzyl-a-D-galactopyranosyl)-a-D-glucopyranoside as an amorphous solid.

### EXAMPLE 53

### Preparation of METHYL 6-O-(2,3,6-TRI-O-BENZYL-4-O-TRIFLUOROMETHYLSULFONYL-a-D-GALACTOPYRANOSYL)-2,3,4-TRI-O-BENZYL-a-D-GLUCOPYRANOSIDE

To a solution of dry pyridine (0.45 ml) in dry methylene chloride (40 ml) cooled to -15 ° C is added trifluoromethane sulfonic anhydride (0.83 ml). The mixture is stirred during 15 min at -10 ° C, then methyl 2,3,4-tri-O-benzyl-6-O-(2,3,6-tri-O-benzyl-a-D-galactopyranosyl)-a-D-glucopyranoside (2.21 g, 2.46 mmol) in methylene chloride (10 ml) is added. The mixture is washed with water. The organic layer is dried over sodium sulfate, filtered and concentrated under reduced pressure so as to afford an oil which will be the expected triflate methyl 6-O-(2,3,6-tri-O-benzyl-4-O-tri-fluoromethylsulfonyl-α-D-galactopyranosyl)-2,3,4-tri-O-benzyl-a-D-glucopyranoside.

### EXAMPLE 54

### Preparation of 2,3,6-TRI-O-BENZYL-1,5-DIDEOXY-N-[2,3,6-TRI-O-BENZYL-4-DEOXY-1-(2,3,4-TRI-O-BENZYL-1-O-METHYL-6-O-α-D-GLUCOPYRANOSYL)-α-D-GLUCOPYRANOSYL]-1,5-IMINO-D-GLUCITOL

A solution of methyl 6-O-(2,3,6-tri-O-benzyl-4-O-trifluoromethylsulfonyl-a-D-galactopyranosyl)-2,3,4-tri-O-benzyl-a-D-glucopyranoside (1.6 g, 1.55 mmol) and 2,3,6-tri-O-benzyl-1,5-dideoxy-1,5-imino-D-glucitol (0.671 g, 1.55 mmol) in ethanol-free chloroform (50 ml) is refluxed under nitrogen during 48 h. The mixture is diluted in methylene chloride and washed successively with a saturated aqueous solution of sodium bicarbonate and saturated brine. The organic layer is dried over sodium sulfate, filtered and concentrated under reduced pressure so as to afford a foam. Flash chromatography on silica gel and elution with a graded mixture of hexane and ethyl acetate will afford the expected amine 2,3,6-tri-O-benzyl-1,5-dideoxy-N-[2,3,6-tri-O-benzyl-4-deoxy-1-(2,3,4-tri-O-benzyl-1-O-methyl-6-O-α-D-glucopyranosyl)-α-D-glucopyranosyl]-1,5-imino-D-glucitolas an amorphous solid.

### EXAMPLE 55

### Preparation of 1,5-DIDEOXY-N-[4-DEOXY-1-(1-O-METHYL-6-O-α-D-GLUCOPYRANOSYL)-α-D-GLUCOPYRANOSYL]-1,5-IMINO-D-GLUCITOL

2,3,6-tri-O-benzyl-1,5-dideoxy-N-[2,3,6-tri-O-benzyl-4-deoxy-1-(2,3,4-tri-O-benzyl-1-O-methyl-6-O-α-D-glucopyranosyl)-a-D-glucopyranosyl]-1,5-imino-D-glucitol (1.2 g, 0.915 mmol) is dissolved in methanol (30 ml). Palladium hydroxyde 20% on charcoal (0.6 g) is added. The mixture is hydrogenated during 4 days at 3 atmospheres. The catalyst is removed by filtration and the solvents are evaporated under reduced pressure. Flash chromatography on silica gel and elution with a graded mixture of chloroform, methanol and water will afford the expected amine 1,5-dideoxy-N-[4-deoxy-l-(1-0-methyl-6-0-a-D-glucopyranosyl)-a-D-glucopyranosyl]-1,5-imino-D-glucitol as an amorphous solid.

### EXAMPLE 56

### Preparation of 2,3,6-TRI-O-BENZYL-4-DEOXY-4-HYDROXYMETHYL-D-GLUCOPYRANOSE

Methyl 2,3,6-tri-O-benzyl-4-deoxy-4-hydroxymethyl-a-D-glucopyranoside (4.78 g, 10 mmol) is dissolved at 0 _{°} C in a 9:1 mixture of trifluoroacetic acid and water (50 ml). The mixture is stirred overnight at 0 ° C. The solvents are evaporated under reduced pressure without heating. The residue is dissolved in ethyl acetate and washed successively with sodium bicarbonate and brine. The organic layer is dried over sodium sulfate, filtered and concentrated under reduced pressure so as to afford an oil. Flash chromatography on silica gel and elution with a graded mixture of ethyl acetate and hexane will afford 2,3,6-tri-O-benzyl-4-deoxy-4-hydroxymethyl-D-glucopyranose as an oil.

### EXAMPLE 57

### Preparation of ACETYL 2,3,6-TRI-O-BENZYL-4-DEOXY-4-ACETYLOXYMETHYL-D-GLUCO-PYRANOSIDE

2,3,6-tri-O-benzyl-4-deoxy-4-hydroxymethyl-D-glucopyranose (5.10 g, 9.30 mmol) is dissolved in dry pyridine (25 ml) and acetic anhydride (5 ml) is added. The mixture is stirred during 24 h at room temperature. The solvent is evaporated under high vacuum. The residue is dissolved in ethyl acetate and washed with water. The organic layer is dried over sodium sulfate, filtered and concentrated under reduced pressure so as to afford the expected diacetate acetyl 2,3,6-tri-O-benzyl-4-deoxy-4-acetyloxymethyl-D-gluco-pyranoside as an oil which is used without purification.

### EXAMPLE 58

### Preparation of 2,3,6-TRI-O-BENZYL-1,4-DIDEOXY-4-ACETYLOXYMETHYL-D-GLUCOPYRANOSYL CHLORIDE

Acetyl 2,3,6-tri-O-benzyl-4-deoxy-4-acetyloxymethyl-D-glucopyranoside (5.10 g, 9.30 mmol) in ether (10 ml) is treated with ethereal hydrogen chloride (0.2 g/ml, 25 ml). The mixture is stirred at room temperature during 48 h. The solvents are evaporated under reduced pressure so as to afford an oil. Flash chromatography on silica gel and elution with a graded mixture of carbon tetrachloride and ethyl acetate will afford 2,3,6-tri-O-benzyl-1,4-dideoxy-4-acetyloxymethyl-D-glucopyranosyl chloride as an oil.

### EXAMPLE 59

### Preparation of METHYL 4-O-(2,3,6-TRI-O-BENZYL-4-DEOXY-4-ACETYLOXYMETHYL-α D-GLUCOPYRANOSYL)-2,3,6-TRI-O-BENZYL-a-D-GLUCOPYRANOSIDE

Ethereal silver perchlorate (0.08 M, 95.8 ml, 7.67 mmol) is added with stirring at -30 ° C to a solution of methyl 2,3,6-tri-O-benzyl-a-D-glucopyranoside (2.592 g, 5.59 mmol), 2,3,6-tri-O-benzyl-1,4-dideoxy-4-ac- etyloxymethyl-D-glucopyranosyl chloride (3.661 g, 6.98 mmol) in ether (20 ml). The mixture is stirred 15 min at -30 °C and silver chloride precipitated. The mixture is filtered through a celite pad, the solids are washed with ether, the filtrate is concentrated under reduced pressure. The residue is dissolved in methylene chloride and the organic layer is washed successively with aqueous sodium thiosulfate and water. The organic layer is dried over sodium sulfate, filtered and concentrated under reduced pressure so as to afford a foam. Flash chromatography on silica gel and elution with a graded mixture of hexane and ethyl acetate will afford methyl 4-O-(2,3,6-tri-O-benzyl-4-deoxy-4-acetyloxymethyl-a-D-glucopyranosyl)-2,3,6-tri-O-benzyl-a-D-glucopyranoside as a foam.

### EXAMPLE 60

### Preparation of METHYL 4-O-(2,3,6-TRI-O-BENZYL-4-DEOXY-4-HYDROXYMETHYL-a-D-GLUCOPYRANOSYL)-2,3,6-TRI-O-BENZYL-a-D-GLUCOPYRANOSIDE

Methyl 4-O-(2,3,6-tri-O-benzyl-4-deoxy-4-acetyloxymethyl-α-D-glucopyranosyl)-2,3,6-tri-O-benzyl-α-D-glucopyranoside (3.19 g, 3.35 mmol) is dissolved in hot toluene (20 ml) and methanol (80 ml) is added, followed by a few drops of 1 M methanolic sodium methoxide. The mixture is stirred at room temperature during 2 h. The reaction mixture is made neutral with Amberlite IR 120 (H⁺) resin, faltered and concentrated under reduced pressure so as to afford methyl 4-O-(2,3,6-tri-O-benzyl-4-deoxy-4-hydroxymethyl-a-D-glucopyranosyl)-2,3,6-tri-0-benzyl-a-D-glucopyranoside as an amorphous solid.

### EXAMPLE 61

### Preparation of METHYL 4-O-(2,3,6-TRI-O-BENZYL-4-DEOXY-4-TRIFLUOROMETHYLSULFONYLOXMETHYL-α-D-GLUCOPYRANOSYL)-2,3,6-TRI-O-BENZYL-α-D-GLUCOPYRANOSIDE

To a solution of dry pyridine (0.6 ml) in dry methylene chloride (50 ml) cooled to -15 ° C is added trifluoromethane sulfonic anhydride (1.12 ml). The mixture is stirred during 15 min at -10 ° C, then methyl 4-O-(2,3,6-tri-O-benzyl-4-deoxy-4-hydroxymethyl-α-D-glucopyranosyl)-2,3,6-tri-O-benzyl-α-D-glucopyranoside (3.049g, 3.35 mmol) in methylene chloride (15 ml) is added. The mixture is washed with water. The organic layer is dried over sodium sulfate, filtered and concentrated under reduced pressure so as to afford an oil which will be the expected triflate methyl 4-O-(2,3,6-tri-O-benzyl-4-deoxy-4-trifluoromethylsulfonyloxymethyl- a-D-glucopyranosyl)-2,3,6-tri-O-benzyl-a-D-glucopyranoside.

### EXAMPLE 62

### Preparation of 2,3,6-TRI-O-BENZYL-1,5-DIDEOXY-N-{[2,3,6-TRI-O-BENZYL-4-DEOXY-1-(2,3,6-TRI-O-BENZYL-4-O-α-D-GLUCOPYRANOSYL)-4-α-D-GLUCOPYRANOSYL]METHYL}1,5-IMINO-D-GLUCITOL

A solution of methyl 4-O-(2,3,6-tri-O-benzyl-4-deoxy-4-trifluoromethylsulfonyloxymethyl-a-D-glucopyranosyl)-2,3,6-tri-O-benzyl-a-D-glucopyranoside (1.82 g, 1.75 mmol) and 2,3,6-tri-O-benzyl-1,5- dideoxy-1,5-imino-D-glucitol (0.758 g, 1.75 mmol) in ethanol-free chloroform (50 ml) is refluxed under nitrogen during 48 h. The mixture is diluted in methylene chloride and washed successively with a saturated solution of sodium bicarbonate and saturated brine. The organic layer is dried over sodium sulfate, filtered and concentrated under reduced pressure so as to afford a foam. Flash chromatography on silica gel and elution with a graded mixture of hexane and ethyl acetate will afford the expected amine 2,3,6-tri-O-benzyl-1,5-dideoxy-N-{[2,3,6-tri-O-benzyl-4-deoxy-1-(2,3,6-tri-O-benzyl-4-O-α-D-glucopyranosyl)-4-α-D-glucopyranosyl]methyl}1,5-imino-D-glucitol as an amorphous solid.

### EXAMPLE 63

### Preparation of 1,5-DIDEOXY-N-{(4-DEOXY-1-(1-O-METHYL-4-O-α-D-GLUCOPYRANOSYL)-4-α-D-GLUCOPYRANOSYL]METHYL}1,5-IMINO-D-GLUCITOL

2,3,6-tri-O-benzyl-1,5-dideoxy-N-{[2,3,6-tri-O-benzyl-4-deoxy-1-(2,3,6-tri-O-benzyl-1-O-methyl-4-O-α-D-glucopyranosyl)-4-α-D-glucopyranosyl]methyl}1,5-imino-D-glucitol (1.3 g, 1.247 mmol) is dissolved in methanol (40 ml). Palladium hydroxyde 20% on charcoal (0.6 g) is added. The mixture is hydrogenated during 4 days at 3 atmospheres. The catalyst is removed by filtration and the solvents are evaporated under reduced pressure. Flash chromatography on silica gel and elution with a graded mixture of chloroform, methanol and water will afford the expected amine 1,5-dideoxy-N-{[4-deoxy-1-(1-O-methyl-4-O-α-D-glucopyranosyl)-4-α-D-glucopyranosyl]methyl}1,5-imino-D-glucitol as an amorphous solid.

### EXAMPLE 64

### Preparation of METHYL 6-O-(2,3,6-TRI-O-BENZYL-4-DEOXY-4-ACETYLOXYMETHYL-a-D-GLUCOPYRANOSYL)-2,3,4-TRI-O-BENZYL-a-D-GLUCOPYRANOSIDE

Ethereal silver perchlorate (0.08 M, 76.7 ml, 6.13 mmol) is added with stirring at -30 ° C to a solution of methyl 2,3,4-tri-O-benzyl-a-D-glucopyranoside (2.074 g, 4.472 mmol), 2,3,6-tri-O-benzyl-1,4-dideoxy-4-ac- etyloxymethyl-D-glucopyranosyl chloride (6.13 mmol) and 2,4,6-trimethylpyridine (0.80 ml, 6.13 mmol) in ether (20 ml). The mixture is stirred 15 min at -30 ° C and silver chloride precipitated. The mixture is filtered through a celite pad, the solids are washed with ether, the filtrate is concentrated under reduced pressure. The residue is dissolved in methylene chloride and the organic layer is washed successively with aqueous sodium thiosulfate and water. The organic layer is dried over sodium sulfate, filtered and concentrated under reduced pressure so as to afford a foam. Flash chromatography on silica gel and elution with a graded mixture of hexane and ethyl acetate will afford methyl 6-O-(2,3,6-tri-O-benzyl-4-deoxy-4-acetylox- ymethyl-a-D-glucopyranosyl)-2,3,4-tri-O-benzyl-a-D-glucopyranoside as a foam.

### EXAMPLE 65

### Preparation of METHYL 6-O-(2,3,6-TRI-O-BENZYL-4-DEOXY-4-HYDROXYMETHYL-a-D-GLUCOPYRANOSYL)-2,3,4-TRI-O-BENZYL-a-D-GLUCOPYRANOSIDE

Methyl 6-O-(2,3,6-tri-O-benzyl-4-deoxy-4-acetyloxymethyl-a-D-glucopyranosyl)-2,3,4-tri-O-benzyl-a-D-glucopyranoside (2.469 g, 2.593 mmol) is dissolved in hot toluene (20 ml) and methanol (80 ml) is added, followed by a few drops of 1 M methanolic sodium methoxide. The mixture is stirred at room temperature during 2 h. The reaction mixture is made neutral with Amberlite IR 120 (H+) resin, filtered and concentrated under reduced pressure so as to afford methyl 6-O-(2,3,6-tri-O-benzyl-4-deoxy-4-hydroxymethyl-a-D-glucopyranosyl)-2,3,4-tri-0-benzyl-a-D-glucopyranoside as an amorphous solid.

### EXAMPLE 66

### Preparation of METHYL 6-O-(2,3,6-TRI-O-BENZYL-4-DEOXY-4-TRIFLUOROMETHYLSULFONYLOX-YMETHYL-a-D-GLUCOPYRANOSYL)-2,3,4-TRI-O-BENZYL-a-D-GLUCOPYRANOSIDE

To a solution of dry pyridine (0.46 ml) in dry methylene chloride (40 ml) cooled to -15 ° C is added trifluoromethane sulfonic anhydride (0.86 ml). The mixture is stirred during 15 min at -10 ° C, then methyl 6-O-(2,3,6-tri-O-benzyl-4-deoxy-4-hydroxymethyl-α-D-glucopyranosyl)-2,3,4-tri-O-benzyl-α-D-glucopyranoside (2.36 g, 2.593 mmol) in methylene chloride (10 ml) is added. The mixture is stirred during 1.5 h at -10 ° C. The reaction mixture is washed with water. The organic layer is dried over sodium sulfate, filtered and concentrated under reduced pressure so as to afford an oil which will be the expected triflate methyl 6-O-(2,3,6-tri-O-benzyl-4-deoxy-4-trifluoromethylsulfonyloxymethyl-a-D-glucopyranosyl)-2,3,4-tri-O-benzyl-a-D-glucopyranoside.

### EXAMPLE 67

### Preparation of 2,3,6-TRI-O-BENZYL-1,5-DIDEOXY-N-{[2,3,6-TRI-O-BENZYL-4-DEOXY-1-(2,3,4-TRI-O-BENZYL-1-O-METHYL-6-O-α-D-GLUCOPYRANOSYL)-4-α-D-GLUCOPYRANOSYL]METHYL}1,5-IMINO-D-GLUCITOL

A solution of methyl 6-O-(2,3,6-tri-O-benzyl-4-deoxy-4-trifluoromethylsulfonyloxymethyl-a-D-glucopyranosyl)-2,3,4-tri-O-benzyl-a-D-glucopyranoside (1.8 g, 1.72 mmol) and 2,3,6-tri-O-benzyl-1, 5- dideoxy-1,5-imino-D-glucitol (0.745 g, 1.72 mmol) in ethanol-free chloroform (50 ml) is refluxed under nitrogen during 48 h. The mixture is diluted in methylene chloride and washed successively with a saturated aqueous solution of sodium bicarbonate and saturated brine. The organic layer is dried over sodium sulfate, filtered and concentrated under reduced pressure so as to afford a foam. Flash chromatography on silica gel and elution with a graded mixture of hexane and ethyl acetate will afford the expected amine 2,3,6-tri-O-benzyl-1,5-dideoxy-N-{[2,3,6-tri-O-benzyl-4-deoxy-1-(2,3,4-tri-O-benzyl-1-O-methyl-6-O-α-D-glucopyranosyl)-4-α-D-glucopyranosyl]methyl}1 ,5-imino-D-glucitol as an amorphous solid.

### EXAMPLE 68

### Preparation of 1,5-DIDEOXY-N-{[4-DEOXY-1-(1-O-METHYL-6-O-α-D-GLUCOPYRANOSYL)-4-α-D-GLUCOPYRANOSYL]METHYL}1,5-IMINO-D-GLUCITOL

2,3,6-tri-O-benzyl-1,5-dideoxy-N-{[2,3,6-tri-O-benzyl-4-deoxy-1-(2,3,4-tri-O-benzyl-1-O-methyl-6-O-α-D-glucopyranosyl)-4-α-D-glucopyranosyl]methyl}1,5-imino-D-glucitol (1.3 g, 1.247 mmol) is dissolved in methanol (30 ml). Palladium hydroxyde 20% on charcoal (0.6 g) is added. The mixture is hydrogenated during 4 days at 3 atmospheres. The catalyst is removed by filtration and the solvents are evaporated under reduced pressure. Flash chromatography on silica gel and elution with a graded mixture of chloroform, methanol and water will afford the expected amine 1,5-dideoxy-N-{[4-deoxy-1-(1-O-methyl-6-O-α-D-glucopyranosyl)-4-α-D-glucopyranosyl]methyl}1,5-imino-D-glucitol as an amorphous solid.

### EXAMPLE 69

### Preparation of 1,5-DIDEOXY-1,5-(6-DEOXY-6-D-GLUCOPYRANOSYL)IMINO-D-GLUCITOL

1,5-dideoxy-1,5-(6-deoxy-I-O-methyl-6-a-D-glucopyranosyl)imino-D-glucitol (0.150 g, 0.442 mmol) is dissolved in a 1:1 mixture of water and trifluoroacetic acid (10 ml). The mixture is stirred during 24 h at 0 _{°} C. The solvents are evaporated under reduced pressure so as to afford a foam Chromatography on Amberlyst A26 OH- form will afford the expected amine 1,5-dideoxy-1,5-(6-deoxy-6-D-glucopyranosyl)-imino-D-glucitol.

### EXAMPLE 70

### Preparation of 5-AZIDO-3,6-DI-0-BENZYL-5-DEOXY-D-GLUCOFURANOSE

The azide 5-azido-3,6-di-O-benzyl-5-deoxy-1,2-0-isopropylidene-a-D-glucofuranoside (U.G. Nayak and R.L. Whisler, J. Org. Chem., 33, 3582 (1968)) (15.02 g, 35.3 mmol) was dissolved at 0 ° C in 100 ml of a 9:1 mixture of trifluoroacetic acid and water. The mixture was stirred at 0 ° C during 2 h. The trifluoroacetic acid was evaporated under reduced pressure at room temperature. The residue was taken with ether and washed with water. The organic layer was dried over sodium sulfate, filtered and concentrated under reduced pressure. Flash chromatography on silica gel and elution with a 1:1 mixture of hexane and ethyl acetate, followed by recrystallization in a mixture of hexane and ethyl acetate afforded the expected compound 5-azido-3,6-di-O-benzyl-5-deoxy-D-glucofuranose.

### EXAMPLE 71

### Preparation of METHYL 5-AZIDO-3,6-DI-0-BENZYL-5-DEOXY-D-GLUCOFURANOSIDE

To a solution of 5-azido-3,6-di-O-benzyl-5deoxy-D-glucofuranose (10.23 g, 26.5 mmol) in methylene chloride (170 ml) was added methanol (11 ml) and boron trifluoride etherate (1.5 ml). The mixture was stirred 24 h at room temperature. The reaction mixture was successively washed with a saturated aqueous solution of sodium bicarbonate and then with brine. The organic layer was dried over sodium sulfate, filtered and concentrated under reduced pressure. Flash chromatography on silica gel and elution with a 1:1 mixture of hexane and ethyl acetate afforded methyl 5-azido-3,6-di-O-benzyl-5-deoxy-D-glucofuranoside as a colorless oil (9.15 g, 85%).

### EXAMPLE 72

### Preparation of METHYL 5-AZIDO-2,3,6-TRI-0-BENZYL-5-DEOXY-D-GLUCOFURANOSIDE

To a suspension of sodium hydride (1.2 g, 27.5 mmol), 55% in mineral oil, washed three times with pentane) in anhydrous tetrahydrofuran (200 ml) was added quickly dropwise the alcohol methyl 5-azido-3,6-di-O-benzyl-5-deoxy-D-gluco-furanoside (9.15 g, 22.9 mmol) in tetrahydrofuran (50 ml) at room temperature and under nitrogen. The mixture was stirred during 3 h at room temperature. The mixture was yellow. Then n-Bu₄ N⁺I- (76 mg, 0.20 mmol) was added followed by benzyl bromide (3.30 ml, 27.5 mmol) added dropwise. The mixture was stirred overnight at room temperature. After hydrolysis with saturated aqueous ammonium chloride tetrahydrofuran was evaporated under reduced pressure. The residue was diluted with water and extracted three times with ether. The organic phase was dried over sodium sulfate. Filtration and evaporation under reduced pressure afforded an oil. Flash chromatography on silica gel and elution with a 20:80 mixture of ethyl acetate and hexane afforded the expected compound methyl 5-azido-2,3,6-tri-O-benzyl-5-deoxy-D-glucofuranoside as a colorless oil (10.88 g, 97%).

### EXAMPLE 73

### Preparation of 5-AZIDO-2,3,6-TRI-O-BENZYL-5-DEOXY-D-GLUCOFURANOSE

Methyl 5-azido-2,3,6-tri-O-benzyl-5-deoxy-D-glucofurano-side (10.8 g, 22.2 mmol) was dissolved at room temperature in tetrahydrofuran (20 ml). The solution was cooled at -10°C and trifluoroacetic acid (120 ml) was added dropwise followed by addition of water (20 ml). The mixture was stirred at 0 ° C during 24 h. The mixture was evaporated under reduced pressure without heating. The residue was taken with ether and washed with water. The organic layer was dried over sodium sulfate, filtered and concentrated under reduced pressure. Flash chromatography on silica gel and elution with a 20:80 mixture of ethyl acetate and hexane afforded 5-azido-2,3,6-tri-O-benzyl-5-deoxy-D-glucofuranose as a colorless oil (9.63 g, 90%).

### EXAMPLE 74

### Preparation of 5-AZIDO-2,3,6-TRI-O-BENZYL-5-DEOXY-D-GLUCONIC ACID-y-LACTONE

To a solution of the lactol 5-azido-2,3,6-tri-O-benzyl-5-deoxy-D-glucofuranose (9.36 g, 20 mmol) in acetone (240 ml) cooled to 0 ° C, Jones' reagent 2 M (11.5 ml) was added dropwise until the color was orange. The excess of Jones' reagent was destroyed with 2-propanol (0.5 ml). The mixture was concentrated under reduced pressure. The residue was taken with water and extracted with ether. The organic phase was dried with sodium sulfate, filtered and concentrated under reduced pressure so as to afford an oil. Flash chromatography on silica gel and elution with a 1:9 mixture of ethyl acetate and hexane afforded the y-lactone 5-azido-2,3,6-tri-O-benzyl-5-deoxy-D-gluconic acid-γ-lactone.

### EXAMPLE 75

### Preparation of 2,3,6-TRI-0-BENZYL-5-DEOXY-D-GLUCONIC ACID-o-LACTAM

To a solution of the lactone 5-azido-2,3,6-tri-O-benzyl-5-deoxy-D-gluconic acid-y-lactone (8.16 g, 17 mmol) in ethanol (180 ml) was added Lindlar catalyst (1.7 g). The mixture was hydrogenated under atmospheric pressure during 24 h. Filtration and evaporation under reduced pressure afforded an oil which was crystallized in a mixture of hexane and ether. The lactam 2,3,6-tri-O-benzyl-5-deoxy-D-gluconic acid-o-lactam was obtained as white crystals (7.4 g, 96%). mp: 85-85.5 ° C.

By substituting those specifically mentioned final products set forth above which are analogous to 1,5- dideoxy-1,5-[(6-deoxy-1-O-methyl-6-a-D-glucopyranosyl)imino]-D-glucitol and by following the teachings of Examples 2 through 6, there will be produced the final compounds of this invention (Formula I) which are analogous to the final compound of Example 6. Similarly, by preparing the appropriately positioned bromo derivative (analogous to methyl-6-bromo-6-deoxy-a-D-glucopyranoside of Example 1) of the above mentioned glycosyl moieties utilized for the condensations reactions with 1-deoxy nojirimycin, and by following the teachings of Example 1, there will be produced the final products analogous to the product of Example 1. these compounds are set forth as follows:
1,5-Dideoxy-4-0(a,D-glucopyranosyl)-1,5-[6,7-dideoxy-7-D-glucoheptopyranosyl)imino]-D-glucitol,
1,5-Dideoxy-4-O(α,D-glucopyranosyl)-1,5-[(1-deoxy-D-fructofuranosyl)imino]-D-glucitol,
1,5-Dideoxy-4-0(a,D-glucopyranosyl)-1,5-[(4-deoxy-4-D-glucopyranosyl)imino]-D-glucitol,
1,5-Dideoxy-4-O(α,D-glucopyranosyl)-N-[6-deoxy-1-(6-O-D-glucopyranosyl)-α-D-glucopyranosyl]-1,5-imino-D-glucitol,
1,5-Dideoxy-4-0(a,D-glucopyranosyl)-N-[6,7-dideoxy-1-(6-0-D-glucopyranosyl)-7-α-D-glucoheptopyranosyl]-1,5-imino-D-glucitol,
1,5-Dideoxy-4-0(a,D-glucopyranosyl)-1,5-[(4-deoxy-4-D-glucopyranosyl)methylimino]-D-glucitol,
1,5-Dideoxy-4-O(α,D-glucopyranosyl)-N-[4-deoxy-1-(4-O-D-glucopyranosyl)-α-D-glucopyranosyl]-1,5-imino-D-glucitol,
1,5-Dideoxy-4-O(α,D-glucopyranosyl)-1,5-{[2(1-D-arabinofuranose)ethyl]imino}-D-glucitol,
1,5-Dideoxy-4-O(α,D-glucopyranosyl)-N-[4-deoxy-1-(6-O-D-glucopyranosyl)-α-D-glucopyranosyl]-1,5-imino-D-glucitol,
1,5-Dideoxy-4-O(α,D-glucopyranosyl)-N-{[4-deoxy-1-(4-O-D-glucopyranosyl)-4-α-D-glucopyranosyl]methyl}-1,5-imino-D-glucitol,
1,5-Dideoxy-4-O(α,D-glucopyranosyl)-N-{[4-deoxy-1-(6-O-D-glucopyranosyl)-4-α-D-glucopyranosyl]methyl}-
1,5-imino-D-glucitol,
1,5-Dideoxy-4-O(α,D-glucopyranosyl)-1,5-[(6-deoxy-1-O-methyl-6-β-D-glucopyranosyl)-imino-D-glucitol,
1,5-Dideoxy-4-O(α,D-glucopyranosyl)-1,5-[(6,7-dideoxy-1-O-methyl-7-β-D-glucoheptopyranosyl)imino]-D-glucitol,
1,5-Dideoxy-4-O(α,D-glucopyranosyl)-1,5-[(1-deoxy-2-O-methyl-β-D-fructofuranosyl)imino]-D-glucitol,
1,5-Dideoxy-4-O(α,D-glucopyranosyl)-1,5-[(4-deoxy-1-O-methyl-4-β-D-glucopyranosyl)imino]-D-glucitol,
1,5-Dideoxy-4-O(α,D-glucopyranosyl)-N-[6-deoxy-1-(1-O-methyl-6-O-β-D-glucopyranosyl)-α-D-glucopyranosyl]-1,5-imino-D-glucitol,
1,5-Dideoxy-4-O(α,D-glucopyranosyl)-N-[6,7-dideoxy-1-(1-O-methyl-6-O-β-D-glucopyranosyl)-7-α-D-glucoheptopyranosyl]-1,5-imino-D-glucitol,
1,5-Dideoxy-4-O(α,D-glucopyranosyl)-1,5-[(4-deoxy-1-O-methyl-4-β-D-glucopyranosyl)methylimino]-D-glucitol,
1,5-Dideoxy-4-O(α,D-glucopyranosyl)-N-[4-deoxy-1-(1-O-methyl-4-O-β-D-glucopyranosyl)-α-D-glucopyranosyl]-1,5-imino-D-glucitol,
1,5-Dideoxy-4-O(α,D-glucopyranosyl)-1,5-{[2-(1-O-methyl-1-β-D-arabinofuranosyl)ethyl]imino}-D-glucitol,
1,5-Dideoxy-4-O(α,D-glucopyranosyl)-N-[4-deoxy-1-(1-O-methyl-6-O-β-D-glucopyranosyl)-α-D-glucopyranosyl]-1,5-imino-D-glucitol,
1,5-Dideoxy-4-O(α,D-glucopyranosyl)-N-{[4-deoxy-1-(1-O-methyl-4-O-β-D-glucopyranosyl)-4-α-D-glucopyranosyl]methyl}-1,5-imino-D-glucitol,
1,5-Dideoxy-4-O(α,D-glucopyranosyl)-N-{[4-deoxy-1-(1-O-methyl-6-O-β-D-glucopyranosyl)-4-α-D-glucopyranosyl]methyl}-1,5-imino-D-glucitol,
1,5-Dideoxy-6-0(a,D-glucopyranosyl)-1,5-[6,7-dideoxy-7-D-glucoheptopyranosyl)imino]-D-glucitol,
1,5-Dideoxy-6-O(α,D-glucopyranosyl)-1,5-[(1-deoxy-D-fructofuranosyl)imino]-D-glucitol,
1,5-Dideoxy-6-0(a,D-glucopyranosyl)-1,5-[(4-deoxy-4-D-glucopyranosyl)imino]-D-glucitol,
1,5-Dideoxy-6-O(α,D-glucopyranosyl)-N-[6-deoxy-1-(6-O-D-glucopyranosyl)-α-D-glucopyranosyl]-1,5-imino-D-glucitol,
1,5-Dideoxy-6-0(a,D-glucopyranosyl)-N-[6,7-dideoxy-1-(6-0-D-glucopyranosyl)-7-α-D-glucoheptopyranosyl]-
1,5-imino-D-glucitol,
1,5-Dideoxy-6-0(a,D-glucopyranosyl)-1,5-[(4-deoxy-4-D-glucopyranosyl)methylimino]-D-glucitol,
1,5-Dideoxy-6-O(α,D-glucopyranosyl)-N-[4-deoxy-1-(4-O-D-glucopyranosyl)-α-D-glucopyranosyl]-1,5-imino-
D-glucitol, 1,5-Dideoxy-6-O(α,D-glucopyranosyl)-1,5-{[2(1-D-arabinofuranose)ethyl]imino}-D-glucitol,
1,5-Dideoxy-6-O(α,D-glucopyranosyl)-N-[4-deoxy-1-(6-O-D-glucopyranosyl)-α-D-glucopyranosyl]-1,5-imino-D-glucitol, 1,5-Dideoxy-6-O(α,D-glucopyranosyl)-N-{[4-deoxy-1-(4-O-D-glucopyranosyl)-4-α-D-glucopyranosyl]methyl}-1,5-imino-D-glucitol,
1,5-Dideoxy-6-O(α,D-glucopyranosyl)-N-{[4-deoxy-1-(6-O-D-glucopyranosyl)-4-α-D-glucopyranosyl]methyl}-
1,5-imino-D-glucitol,
1,5-Dideoxy-6-O(α,D-glucopyranosyl)-1,5-[(6-deoxy-1-O-methyl-6-β-D-glucopyranosyl)-imino-D-glucitol,
1,5-Dideoxy-6-O(α,D-glucopyranosyl)-1,5-[(6,7-dideoxy-1-O-methyl-7-β-D-glucoheptopyranosyl)imino]-D-glucitol,
1,5-Dideoxy-6-O(α,D-glucopyranosyl)-1,5-[(1-deoxy-2-O-methyl-β-D-fructofuranosyl)imino]-D-glucitol,
1,5-Dideoxy-6-O(α,D-glucopyranosyl)-1,5-[(4-deoxy-1-O-methyl-4-β-D-glucopyranosyl)imino]-D-glucitol,
1,5-Dideoxy-6-O(α,D-glucopyranosyl)-N-[6-deoxy-1-(1-O-methyl-6-O-β-D-glucopyranosyl)-α-D-glucopyranosyl]-1,5-imino-D-glucitol,
1,5-Dideoxy-6-O(α,D-glucopyranosyl)-N-[6,7-dideoxy-1-(1-O-methyl-6-O-β-D-glucopyranosyl)-7-α-D-glucoheptopyranosyl]-1,5-imino-D-glucitol,
1,5-Dideoxy-6-O(α,D-glucopyranosyl)-1,5-[(4-deoxy-1-O-methyl-4-β-D-glucopyranosyl)methylimino]-D-glucitol,
1,5-Dideoxy-6-O(α,D-glucopyranosyl)-N-[4-deoxy-1-(1-O-methyl-4-O-β-D-glucopyranosyl)-α-D-glucopyranosyl]-1,5-imino-D-glucitol,
1,5-Dideoxy-6-O(α,D-glucopyranosyl)-1,5-{[2-(1-O-methyl-1-β-D-arabinofuranosyl)ethyl]imino}-D-glucitol, 1,5-Dideoxy-6-O(α,D-glucopyranosyl)-N-[4-deoxy-1-(1-O-methyl-6-O-β-D-glucopyranosyl)-α-D-glucopyranosyl]-1,5-imino-D-glucitol,
1,5-Dideoxy-6-O(α,D-glucopyranosyl)-N-{[4-deoxy-1-(1-O-methyl-4-0-β-D-glucopyranosyl)-4-α-D-glucopyranosyl]methyl}-1 ,5-imino-D-glucitol,
1,5-Dideoxy-6-O(α,D-glucopyranosyl)-N-{[4-deoxy-1-(1-O-methyl-6-O-β-D-glucopyranosyl)-4-α-D-glucopyranosyl]methyl}-1 ,5-imino-D-glucitol.

Enzymes which catalyze the hydrolysis of complex carbohydrates, e.g. a-glycosidases, convert nonab- sorbable carbohydrates into absorbable sugars. The rapid action of these enzymes, particularly following the intake of high levels of carbohydrates, lead to acute high levels in blood glucose which, in the case diabetics, lead to undesirable manifestations, thus it has been a long-sought goal to find compounds which will obviate the hyperglycemia caused by dietary improprieties. Similarly, in the case of obesity the control of high levels of blood glucose, with its subsequent conversion to fat, caused by the catalysis of carbohydrates has inspired the quest for compounds which will obviate the problems associated with dietary improprieties.

The compounds of this invention (I) are potent and long-lasting inhibitors of a-glucosidase and, by standard laboratory methods for determining serum glucose levels, are shown to be useful for the treatment of disease states caused by the underutilization and/or overproduction of serum glucose without adversely affecting the rate of transport across cell membranes. Thus, the compounds are useful in the treatment of diabetes and obesity.

In the practice of this invention, an effective amount of a compound of this invention is that amount required to reduce the amount of serum glucose (relative to a control) following the ingestion of carbohydrates convertible to absorbable glucose. The specific dosage for the treatment of any specific patient suffering from either disease state will depend upon such factors as size, type and age of the patient as well as the severity of the disease state, all of which are factors normally familiar to and considered by the attending diagnostician treating the patient. Generally, the compounds are to be administered orally at a dose of 0.2 to 20 milligrams per kilogram of body weight (MPK) with a dose of 0.5 to 5 MPK being preferred. The compounds preferable are to be administered orally at mealtimes in single or multiple unit doses containing 25 mg to 250 mg. Of course, in the treatment of obesity, the term includes the practice of the disease as well as continued administration of dose regimens suitable for the maintenance of the desired weight for the patient.

It is also to be found that the compounds of the instant invention (I) will exert an inhibitory effect on glycosidase enzymes that are essential for elaboration of the final structure of the oligosaccharide side- chains of glycoproteins, particularly the HIV (gp 120) glycoprotein. Suitable assay techniques, e.g. syncytial formation, the reverse transcriptase assay, immunofluorescence tests and election microscopy, may be used to evaluate the effects on HIV viral growth and for determining dose regimens. Anti-viral effects may be confirmed by immunofluorescence with serum for virally infected patients. In the treatment of the HIV related disease states, as well as other retroviral glycoprotein-related disease states, unlike the treatment of diabetes and obesity, the compounds of this invention may be administered by parenteral means; specific doses being within the above stated dose range for treatment of diabetes and obesity.

In practicing the end-use application of the compounds of this invention, the compounds are preferably incorporated in a pharmaceutical formulation comprising a pharmaceutical carrier in admixture with a compound of this invention. The term "pharmaceutical carrier" refers to known pharmaceutical excipients useful in formulating pharmaceutically active compounds for internal administration to animals, and which are substantially nontoxic and nonsensitizing under conditions of use. The compositions can be prepared by known techniques for the preparation of tablets, capsules, elixirs, syrups, emulsions, dispersions and wettable and effervescent powders, and can contain suitable excipients known to be useful in the preparation of the particular type of composition desired. Suitable pharmaceutical carriers and formulation techniques are found in standard texts, such as Remington's Pharmaceutical Sciences, Mack Publishing Company, Easton, Pa.

## Claims (Claims for the following Contracting State(s) : AT, BE, CH, LI, DE, DK, FR, GB, GR, IT, LU, NL, SE)

1. A compound of the formula 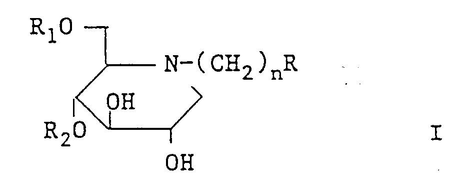 and the pharmaceutically acceptable acid addition salts thereof wherein
n is zero, 1 or 2,
R is a glycosyl moiety containing 1 to 3 hexose or pentose units, said units optionally bearing an ether or acyl radical at the anomeric carbon atom of the terminal hexose or pentose unit, and one of R₁ and R₂ is H and the other is a-D-glucopyranosyl.

2. A compound of Claim 1 wherein R₁ is a-D-glucopyranosyl and R₂ is H.

3. A compound of Claim 1 wherein R₂ is a-D-glucopyranosyl and R₁ is H.

4. A compound of Claim 1 wherein n is one.

5. A compound of Claim 1, said compound being 1,5-dideoxy-4-0-(a-D-glucopyranosyl)-1,5-[(6-deoxy-l-O-methyl-6-a-D-glucopyranosyl)imino]-D-glucitol.

6. A pharmaceutical composition comprising the compound according to any one of claims 1 through 5 and an acceptable pharmaceutical carrier.

7. A compound according to any one of claims 1 through 5 for use as a medicament.

8. The use of a compound according to any one of claims 1 through 5 for the manufacture of a medicament for treating diabetes.

9. The use of a compound according to any one of claims 1 through 5 for the manufacture of a medicament for treating obesity.

10. A process for preparing a compound of the formula

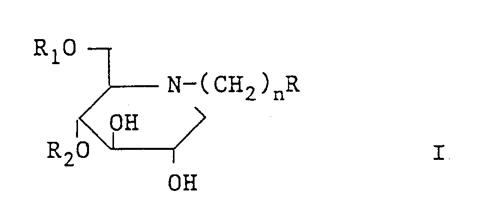

and the pharmaceutically acceptable acid addition salts thereof wherein n is zero, 1 or 2,
R is a glycosyl moiety containing 1 to 3 hexose or pentose units, said units optionally bearing an ether or acyl radical at the anomeric carbon atom of the terminal hexose or pentose unit, and one of R₁ and R₂ is H and the other is a-D-glucopyranosyl,
which comprises the reactions
(a) condensing a compound of the formula 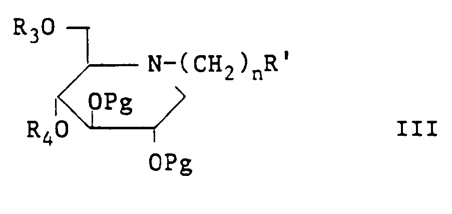 wherein n is zero, one or two, R' is a glycosyl moiety as defined for R in Formula I wherein its OH radicals bear a hydroxy-protecting group, Pg is a hydroxy-protecting group, and one of R₃ and R₄ is a hydroxy-protecting group and the other is H, with a compound of the formula 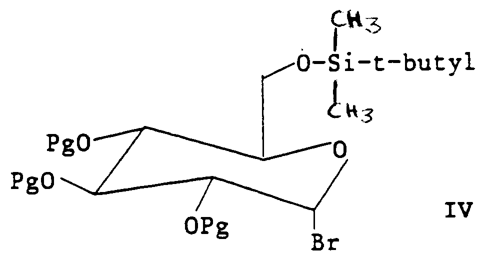 to produce a compound of the formulae 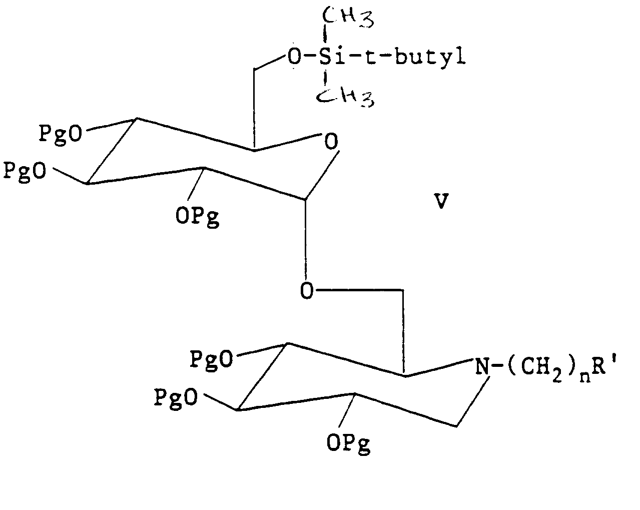 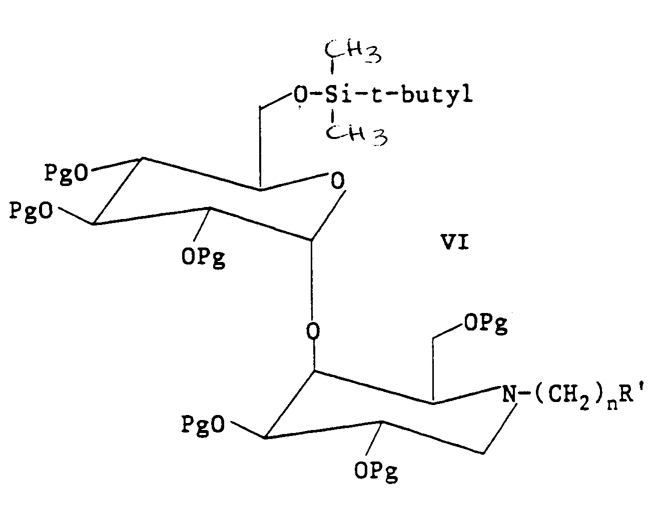 followed by the removal of any hydroxy-protecting groups and, if necessary, neutralizing any ammonium salts, and
(b) condensing a compound of the formula 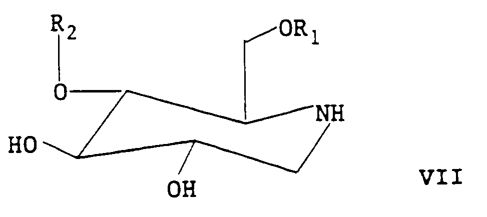 wherein one of R₁ and R₂ is H and the other is an a-D-glucopyranosyl moiety with a compound of the formula X-(CH₂)ₙR wherein X is bromo, n is zero, one or two, and R is as defined for Formula I , said reactions (a) and (b) producing compounds of the formula 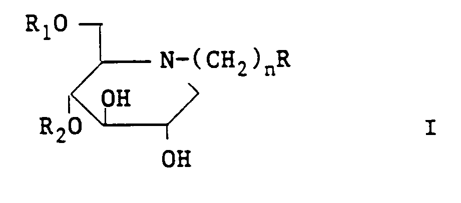 wherein R, Ri, R₂ and n are as defined in Formula I , and optionally converting said compounds to their pharmaceutically acceptable salts.

## Claims (Claims for the following Contracting State(s) : ES)

1. A process for preparing a compound of the formula : 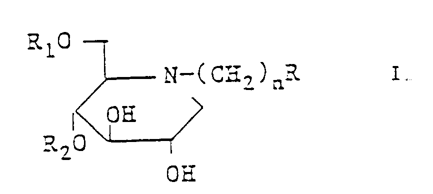 and the pharmaceutically acceptable acid addition salts thereof wherein
- n is zero, 1 or 2,
- R is a glycosyl moiety containing 1 to 3 hexose or pentose units, said units optionally bearing an ether or acyl radical at the anomeric carbon atom of the terminal hexose or pentose unit, and one of R₁ and R₂ is H and the other is a-D- glucopyranosyl, which comprises the reactions of :
(a) condensing a compound of the formula : 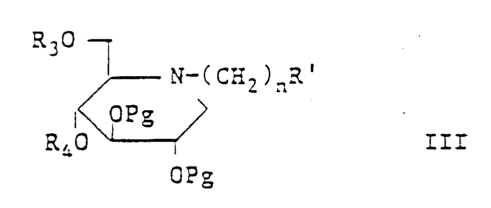 wherein n is zero, one or two, R' is a glycosyl moiety as defined for R in Formula I wherein its OH radicals bear a hydroxy-protecting group, Pg is a hydroxy-protecting group, an one of R₃ and R₄ is a hydroxy-protecting group and the other is H, with a compound of the formula : 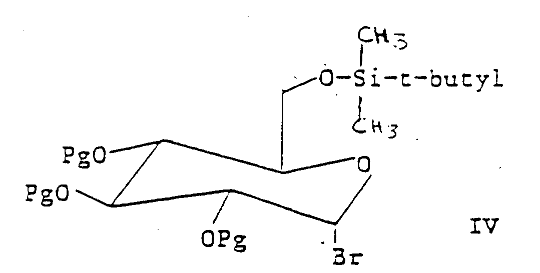 to produce a compound of the formulae : 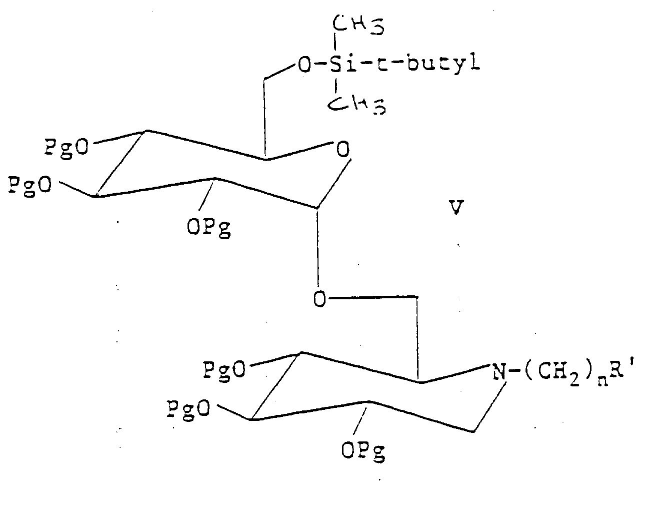 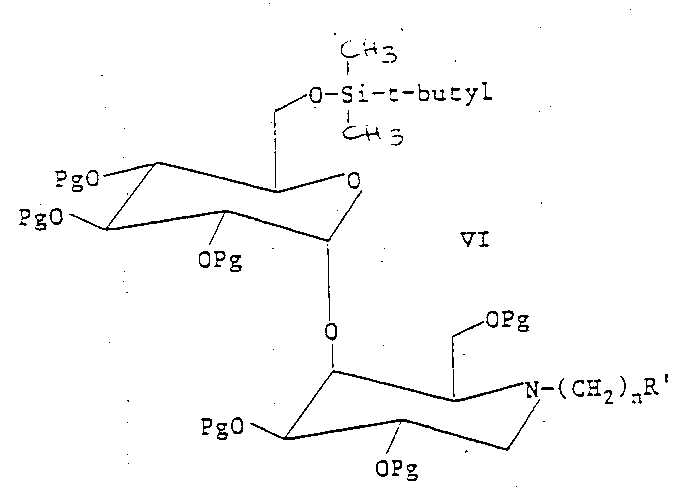 followed by the removal of any hydroxy-protecting groups and, if necessary, neutralizing any ammonium salts, and
(b) condensing a compound of the formula 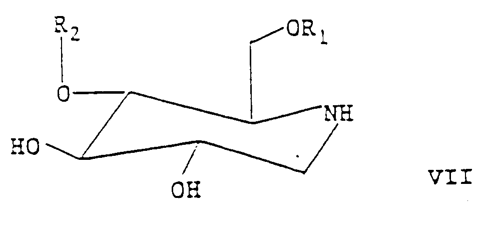 wherein one of R₁ and R₂ is H and the other is an a-D-glucopyranosyl moiety with a compound of formula X-(CH₂)ₙR wherein X is bromo, n is zero, one or two, and R is as defined for Formula I, said reactions (a) and (b) producing compounds of the formula : 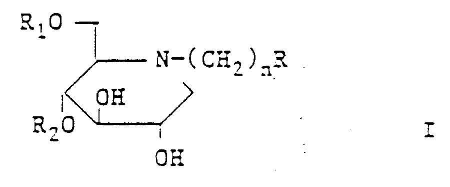 wherein R, Ri, R₂ and n are as defined in Formula II, and optionally converting said compounds to their pharmaceutically acceptable salts.

2. A process of claim 1 wherein R₁ is a-D-glucopyranosyl and R₂ is H.

3. A process of claim 1 wherein R₂ is a-D-glucopyranosyl and R₁ is H.

4. A process of claim 1 wherein n is one.

5. A process of claim 1, which comprises the reaction of 1,5-dideoxy-4-O-(α-D-glucopyranosyl)-1,5-imino-D-glucitol with methyl 6-bromo-6-deoxy-a-D-glucopyranoside to yield the 1,5-dideoxy-4-O-(a-D-glucopyranosyl)-1,5-[(6-deoxy-1-O-methyl-6-α-D-glucopyranosyl)imino]-D-glucitol.

6. The use of a compound obtained according to the process of to any one of claims 1 through 5 for the manufacture of a medicament for treating diabetes.

7. The use of a compound obtained according to the process of any one of claims 1 through 5 for the manufacture of a medicament for treating obesity.

## Patentansprüche (Patentansprüche für folgende(n) Vertragsstaat(en) : AT, BE, CH, LI, DE, DK, FR, GB, GR, IT, LU, NL, SE)

1. Verbindung der Formel 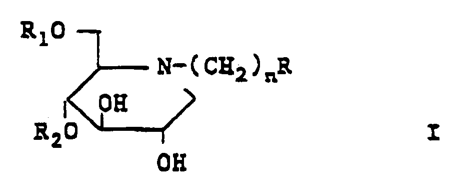 sowie deren pharmazeutisch verträgliche Säureadditionssalze, wobei
n den Wert null, 1 oder 2 hat,
R einen Glycosylrest mit 1 bin 3 Hexose- oder Pentoseeinheiten darstellt, wobei die Einheiten gegebenenfalls einen Ether- oder Acylrest an dem anomeren Kohlenstoffatom dar endständigen Hexose- oder Pentoseeinheit tragen können, und einer der Reste R₁ und R₂ ein Wasserstoffatom und der andere einen a-D-Glucopyranosylrest darstellt.

2. Verbindung nach Anspruch 1, wobei R₁ einen a-D-Glucopyranosylrest und R₂ ein Wasserstofatom darstellt.

3. Verbindung nach Anspruch 1, wobei R₂ einen a-D-Glucopyranosylrest und R₁ ein Wasserstoffatom darstellt.

4. Verbindung nach Anspruch 1, wobei n den Wert eins hat.

5. Verbindung nach Anspruch 1, nämlich 1,5-Dideoxy-4-O-(a-D-glucopyranosyl)-1,5-[(6-deoxy-1-O-methyl-6-a-D-glucopyranosyl)imino]-D-glucitol.

6. Arzneimittel, umfassend die Verbindung nach einem der Ansprüche 1 bis 5 und einen verträglichen pharmazeutischen Träger.

7. Verbindung nach einem der Ansprüche 1 bis 5 zur Verwendung als ein Arzneimittel.

8. Verwendung einer Verbindung nach eine der Ansprüche 1 bis 5 bei der Herstellung eines Arzneimittels zur Behandlung von Diabetes.

9. Verwendung einer Verbindung nach einem der Ansprüche 1 bis 5 bei der Herstellung eines Arzneimittels zur Behandlung von Fettleibigkeit.

10. Verfahren zur Herstellung einer Verbindung der Formel 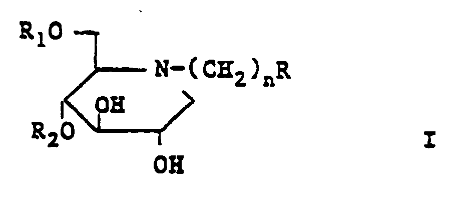 sowie deren pharmazeutisch verträglichen Säureadditionssalze, wobei
n den Wert null, 1 oder 2 hat,
R einen Glycosylrest mit 1 bis 3 Hexose- oder Pentoseeinheiten darstellt, wobei die Einheiten gegebenenfalls einen Ether- oder Acylrest an dem anomeren Kohlenstoffatom der endständigen Hexose- oder Pentoseeinheit tragen können, und einer der Reste R₁ und R₂ ein Wasserstoffatom und der andere einen a-D-Glucopyranosylrest darstellt, umfassend die Umsetzungen
(a) Kondensieren einer Verbindung der Formel 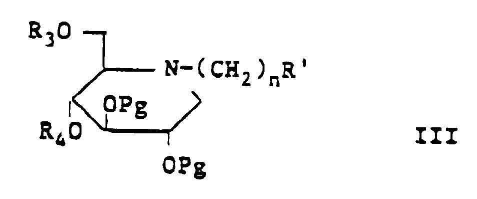 in der n den Wert null, eins oder zwei hat, R' einen wie für R in Formel I definierten Glycosylrest darstellt, wobei dessen OH-Reste eine Hydroxyschutzgruppe tragen, Pg eine Hydroxyschutzgruppe darstellt und einer der Reste R₃ und R₄ eine Hydroxyschutzgruppe und der andere ein Wasserstoffatom darstellt,
mit einer Verbindung der Formel 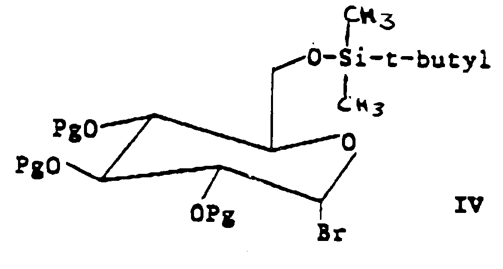 wobei eine Verbindung der Formeln 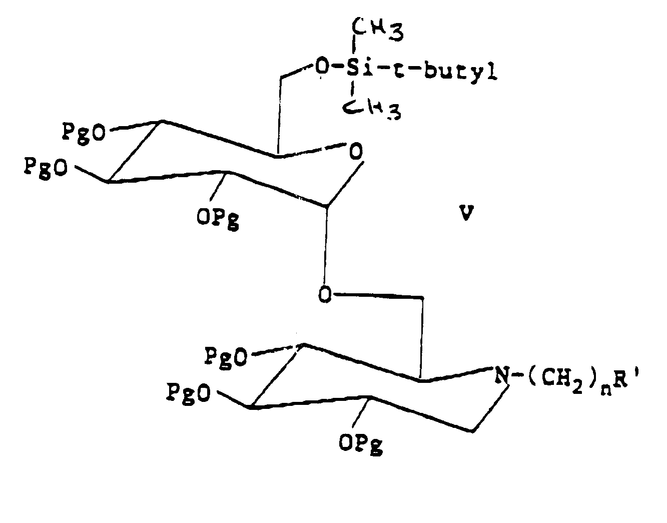 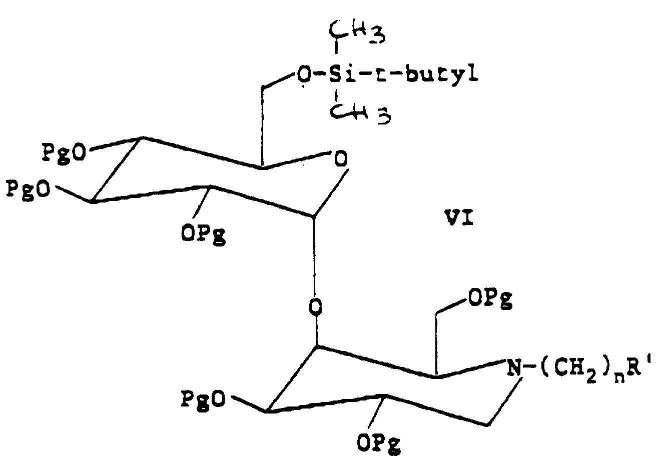 hergestellt wird,
gefolgt von der Entfernung der Hydroxyschutzgruppen und, falls nötig, neutralisieren der Ammoniumsalze, und
(b) Kondensieren einer Verbindung der Formel 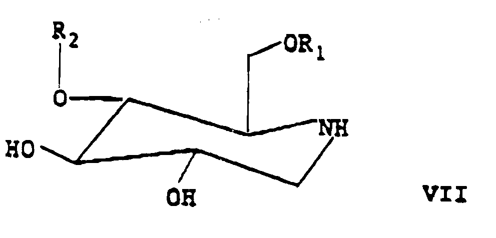 in der einer der Reste R₁ und R₂ ein Wasserstoffatom und der andere einen a-D-Glucopyranosylrest darstellt, mit einer Verbindung der Formel X-(CH₂)ₙR, in der X ein Bromatom darstellt, n den Wert null, eins oder zwei hat und R wie für Formel I definiert ist,
wobei die Umsetzungen (a) und (b) Verbindungen der Formel 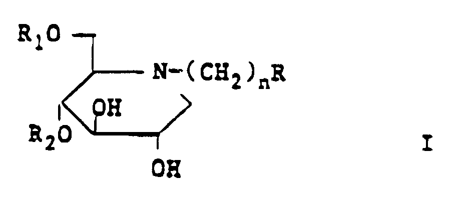 ergeben, wobei R, Ri, R₂ und n wie für Formel I definiert sind, und gegebenenfalls Überführen der Verbindungen in ihre pharmzeutisch verträglichen Salze.

## Patentansprüche (Patentansprüche für folgende(n) Vertragsstaat(en) : ES)

1. Verfahren zur Herstellung einer Verbindung der Formel

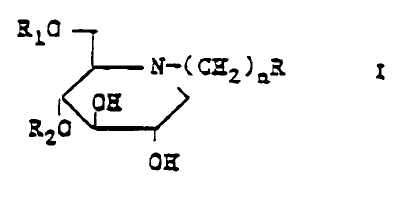

sowie deren pharmazeutisch verträgliche Säureadditionssalze,

wobei
n den Wert null, 1 oder 2 hat,
R einen Glycosylrest mit 1 bis 3 Hexose- oder Pentoseeinheiten darstellt, wobei die Einheiten gegebenenfalls einen Ether- oder Acylrest an dem anomeren Kohlenstoffatom der endständigen Hexose- oder Pentoseeinheit tragen können, und einer der Reste R₁ und R₂ ein Wasserstoffatom und der andere einen a-D-Glucopyranosylrest darstellt, umfassend die Umsetzungen
(a) Kondensieren einer Verbindung der Formel 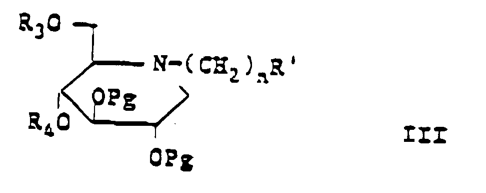 in der n den Wert null, eins oder zwei hat, R' einen wie für R in Formel I definierten Glycosylrest darstellt, wobei dessen OH-Reste eine Hydroxyschutzgruppe tragen, Pg eine Hydroxyschutzgruppe darstellt und einer der Reste R₃ und R₄ eine Hydroxyschutzgruppe und der andere ein Wasserstoffatom darstellt,
mit einer Verbindung der Formel 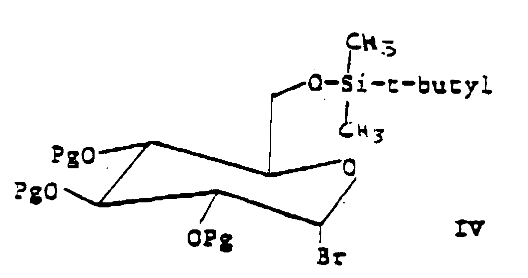 wobei eine Verbindung der Formeln 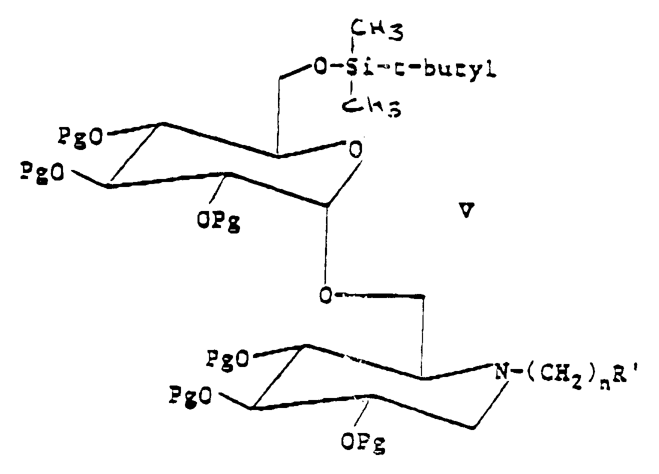 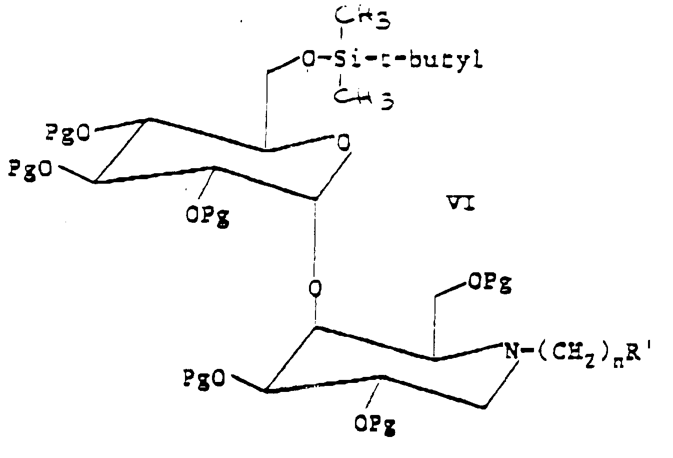 hergestellt wird,
gefolgt von der Entfernung der Hydroxyschutzgruppen und, falls nötig, neutralisieren der Ammoniumsalze, und
(b) Kondensieran einer Verbindung dar Formel 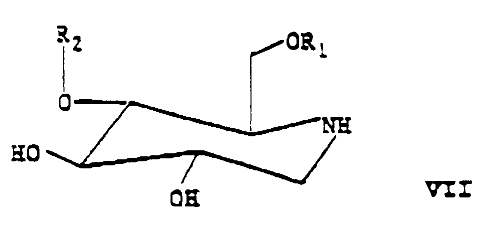 in der einer der Reste R₁ und R₂ ein Wasserstoffatom und der andere einen a-D-Glucopyranosylrest darstellt, mit einer Verbindung der Formel X-(CH₂)ₙR, in der X ein Bromatom darstellt, n den Wert null, eins oder zwei hat und R wie für Formel I definiert ist,
wobei die Umsetzungen (a) und (b) Verbindungen der Formel 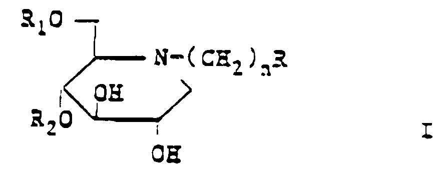 ergeben, wobei R, Ri, R₂ und n wie für Formel I definiert sind, und gegebenenfalls Überführen der Verbindungen in ihre pharmzeutisch verträglichen Salze.

2. Verfahren nach Anspruch 1, wobei R₁ einen a-D-Glucopyranosylrest und R₂ ein Wasserstoffatom darstellt.

3. Verfahren nach Anspruch 1, wobei R₂ einen a-D-Glucopyranosylrest und R₁ ein Wasserstoffatom darstellt.

4. Verfahren nach Anspruch 1, wobei n den Wert eins hat.

5. Verfahren nach Anspruch 1, umfassend die Umsetzung von 1,5-Dideoxy-4-O-(α-D-glucopyranosyl)-1,5- imino-D-glucitol mit Methyl-6-brom-6-deoxy-a-D-glucopyranosid, wobei das 1,5-Dideoxy-4-0-(a-D-glucopyranosyl)-1,5-[(6-deoxy-1-0-methyl-6-a-D-glucopyranosyl)imino]-D-glucitol erhalten wird.

6. Verwendung einer nach einem der Ansprüche 1 bis 5 erhaltenen Verbindung bei der Herstellung eines Arzneimittels zur Behandlung von Diabetes.

7. Verwendung einer nach einem der Ansprüche 1 bis 5 erhaltenen Verbindung bei der Herstellung eines Arzneimittels zur Behandlung von Fettleibigkeit.

## Revendications (Revendications pour l'(les) Etat(s) contractant(s) suivant(s) : suivants : AT, BE, CH, LI, DE, DK, FR, GB, GR, IT, LU, NL, SE)

1. Composé de formule : 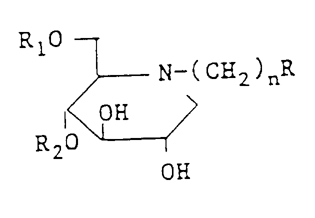 et ses sels d'addition d'acides pharmaceutiquement acceptables, dans laquelle n est égal à 0, 1 ou 2, R est un reste glycosyle contenant 1 à 3 unités hexose ou pentose, lesdites unités portant facultativement un radical éther ou acyle sur l'atome de carbone anomère de l'unité hexose ou pentose finale, et l'un des restes R₁ et R₂ est H et l'autre est un reste a-D-glucopyrannosyle.

2. Composé selon la revendication 1, dans lequel R₁ est un reste a-D-glucopyrannosyle et R₂ est H.

3. Composé selon la revendication 1, dans lequel R₂ est un reste a-D-glucopyrannosyle et R₁ est H.

4. Composé selon la revendication 1, dans lequel n est égal à 1.

5. Composé selon la revendication 1, qui est le 1,5-didésoxy-4-O-(a,D-glucopyrannosyl)-1,5-[(6-désoxy-1-O-méthyl-6-a,D-glucopyrannosyl)imino-D-glucitol.

6. Composition pharmaceutique, comprenant le composé selon l'une quelconque des revendications 1 à 5 et un support acceptable en pharmacie.

7. Composé selon l'une quelconque des revendications 1 à 5, pour l'utilisation comme médicament.

8. Utilisation d'un composé selon l'une quelconque des revendications 1 à 5, pour la fabrication d'un médicament pour le traitement du diabète.

9. Utilisation d'un composé selon l'une quelconque des revendications 1 à 5, pour la fabrication d'un médicament pour le traitement de l'obésité.

10. Procédé pour la préparation d'un composé de formule :

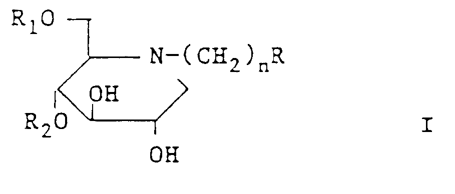

et de ses sels d'addition d'acides pharmaceutiquement acceptables, dans laquelle n est égal à 0, 1 ou 2,
R est un reste glycosyle contenant 1 à 3 unités hexose ou pentose, lesdites unités portant facultativement un radical éther ou acyle sur l'atome de carbone anomère de l'unité hexose ou pentose finale, et l'un des restes R₁ et R₂ est H et l'autre est un reste a-D-glucopyrannosyle,
qui comprend les réactions
(a) de condensation d'un composé de formule : 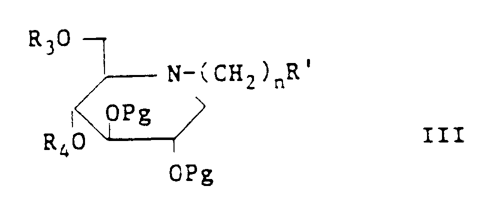 dans laquelle n est égal à zéro, un ou deux, R' est un reste glycosyle tel que défini pour R dans la formule I, dans lequel ses radicaux OH portent un groupe hydroxy-protecteur, Pg est un groupe hydroxy-protecteur et l'un des restes R₃ et R₄ est un groupe hydroxy-protecteur et l'autre est H, avec un composé de formule : 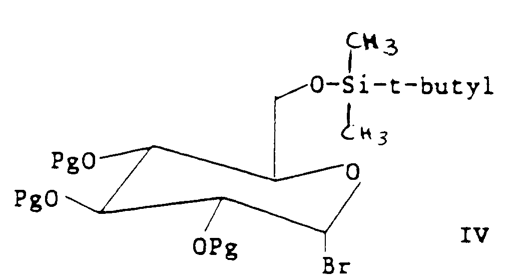 pour produire un composé répondant à l'une des formules : 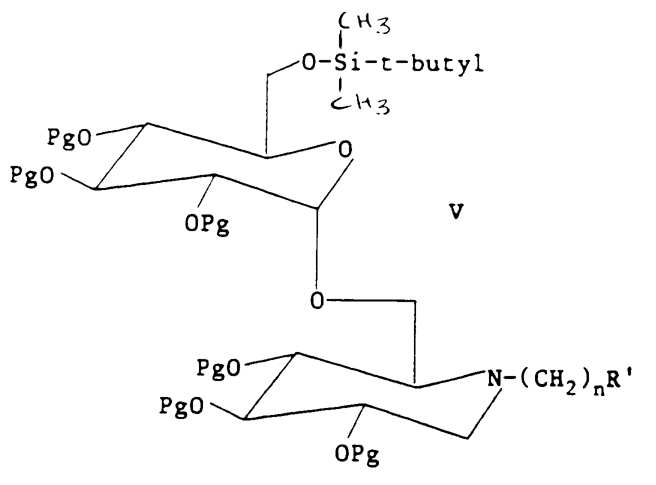 et 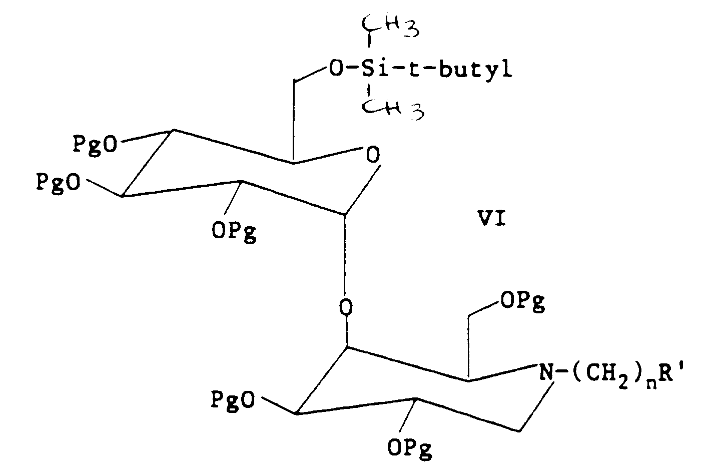 suivie de séparation des groupes hydroxy-protecteurs éventuels et, si nécessaire, de neutralisation des sels d'ammonium éventuels, et
(b) de condensation d'un composé de formule : 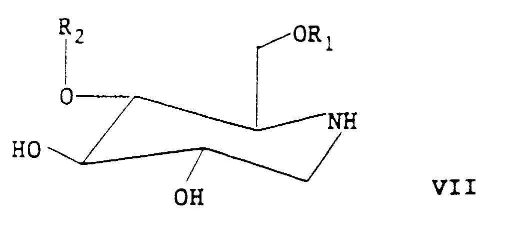 dans laquelle l'un des restes R₁ et R₂ est H et l'autre est un reste a-D-glucopyrannosyle avec un composé de formule X-(CH₂)ₙ-R
dans laquelle X est un atome de brome, n est égal à zéro, un ou deux et R est tel que défini pour la formule 1 ,
lesdites réactions (a) et (b) produisant des composés de formule : 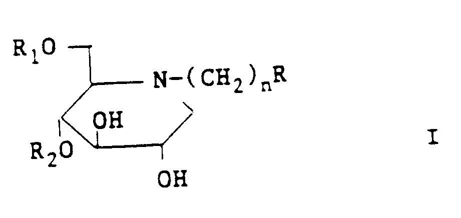 dans laquelle R, Ri, R₂ et n sont tels que définis dans la formule 1 , et facultativement, de transformation desdits composés en leurs sels pharmaceutiquement acceptables.

## Revendications (Revendications pour l'(les) Etat(s) contractant(s) suivant(s) : ES)

1. Procédé pour la préparation d'un composé de formule : 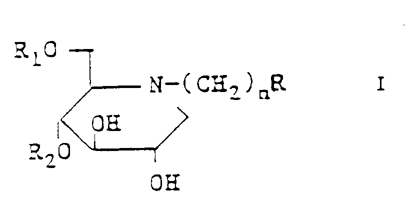 et des ses sels d'addition d'acides pharmaceutiquement acceptables, dans laquelle n est égal à 0,1 ou 2,
R est un reste glycosyle contenant 1 à 3 unités hexose ou pentose, lesdites unités portant facultativement un radical éther ou acyle sur l'atome de carbone anomère de l'unité hexose ou pentose finale, et l'un des restes R₁ et R₂ est H et l'autre est un reste a-D-glucopyrannosyle,
qui comprend les réactions
(a) de condensation d'un composé de formule : 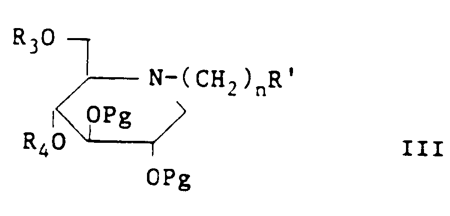 dans laquelle n est égal à zéro, un ou deux, R' est un reste glycosyle tel que défini pour R dans la formule I, dans lequel ses radicaux OH portent un groupe hydroxy-protecteur, Pg est un groupe hydroxy-protecteur et l'un des restes R₃ et R₄ est un groupe hydroxy-protecteur et l'autre est H, avec un composé de formule: 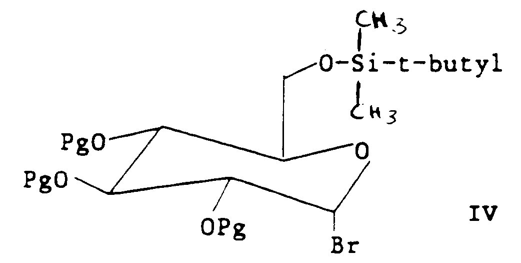 pour produire un composé répondant à l'une des formules : 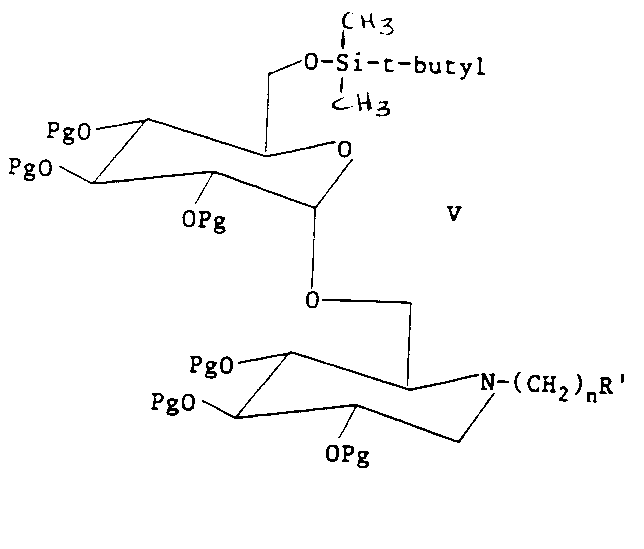 et 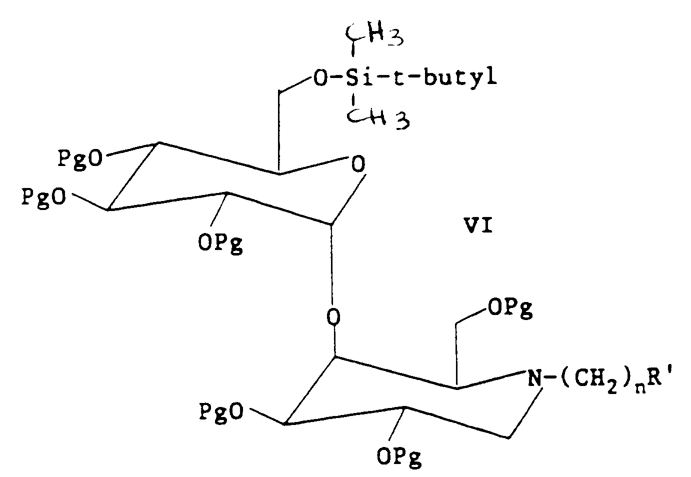 suivie de séparation des groupes hydroxy-protecteurs éventuels et si, nécessaire, de neutralisation des sels d'ammonium éventuels, et
(b) de condensation d'un composé de formule : 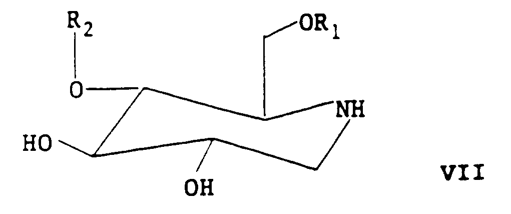 dans laquelle d'un des restes R₁ et R₂ est H et l'autre est un reste a-D-glucopyrannosyle
avec un composé de formule X-(CH₂)ₙ -R dans laquelle X est un atome de brome, n est égal à zéro, un ou deux et R est tel que défini dans la formule 1 , lesdites réactions (a) et (b) produisant des composés de formule : 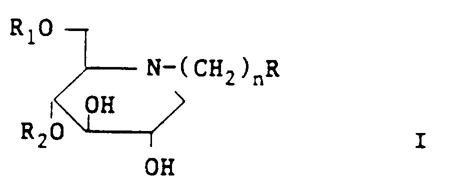 dans laquelle R, Ri, et R₂ et n sont tels que définis dans la formule 1 , et facultativement de transformation desdits composés en leurs sels pharmaceutiquement acceptables.

2. Procédé selon la revendication 1, dans lequel R₁ est un reste a-D-glucopyranosyle et R₂ est l'hydrogène.

3. Procédé selon la revendication 1, dans lequel R₁ est un reste a-D-glucopyranosyle et R₂ est l'hydrogène.

4. Procédé selon la revendication 1, dans lequel n est 1.

5. Procédé selon la revendicationl qui comprend la réaction du 1,5-didéoxy-4-0-(a, D-glucopyrannosyl)-1,5-imino-D-glucitol avec le méthyl 6-bromo-6-déoxy-a-D-glucopyranoside pour donner le 1,5-didéoxy-4-O-(α,D-glucopyranosyl)-1,5-[(6-déoxy-1-O-méthyl-6-α-D-glucopyranosyl)imino]-D-glucitol.

6. Utilisation d'un composé obtenu par le procédé selon l'une quelconque des revendications 1 à 5 pour la fabrication d'un médicament pour le traitement du diabète.

7. Utilisation d'un composé obtenu par le procédé selon l'une quelconque des revendications 1 à 5 pour la fabrication d'un médicament pour le traitement de l'obésité.
